# FASCICULE DE BREVET EUROPEEN

(11) **EP 3 200 842 B1**
(45) Date de publication et mention de la délivrance du brevet: **12.02.2020**
(21) Numéro de dépôt: 15779024.7
(22) Date de dépôt: 28.09.2015
(51) Int. Cl.: A61L 27/56, A61L 27/44

(54) **IMPLANT A POROSITE VARIABLE EN UN MATERIAU HYBRIDE**
HYBRIDMATERIALIMPLANTAT MIT VARIABLER POROSITÄT
HYBRID MATERIAL IMPLANT HAVING VARIABLE POROSITY

(30) Priorité: 29.09.2014 FR 1459209
(43) Date de publication de la demande: 09.08.2017
(73) Titulaire: UNIVERSITE CLERMONT AUVERGNE, 63000 Clermont-Ferrand (FR); Centre National de la Recherche Scientifique, 75016 Paris (FR)
(72) Inventeur: LAO, Jonathan Claude Alexandre, F-63000 Veyre-Monton (FR); JALLOT, Edouard Daniel Albert, F-63360 Saint-Beauzire (FR); DIEUDONNE, Xavier, F-63122 Ceyrat (FR)
(74) Mandataire: Gevers & Orès
(86) Numéro de dépôt international: PCT/IB2015/057420
(87) Numéro de publication internationale: WO 2016/051326

(56) Documents cités:
- WO-A2-2013/023064
- JOAQUÍN RÓDENAS-ROCHINA ET AL: "Comparative study of PCL-HAp and PCL-bioglass composite scaffolds for bone tissue engineering", JOURNAL OF MATERIALS SCIENCE: MATERIALS IN MEDICINE, vol. 24, no. 5, 1 mai 2013 (2013-05-01), pages 1293-1308, XP055132431, ISSN: 0957-4530, DOI: 10.1007/s10856-013-4878-5

## Description

L'invention concerne un matériau d'implant pour le comblement de défauts osseux, la régénération osseuse et l'ingénierie tissulaire de l'os, un implant comprenant ce matériau, et des procédés de fabrication d'un tel implant.

Le vieillissement global de la population et les troubles du système ostéo-articulaire qui l'accompagnent rendent nécessaire le développement de matériaux de substitution des tissus osseux de haute performance. 18 milliards d'euros de frais de santé sont en effet dépensés chaque année en France pour les maladies du système ostéo-articulaire et dentaires, les troubles musculo-squelettiques sont les pathologies professionnelles les plus répandues dans les pays industrialisés, tandis que l'ostéoporose se développe chez les patients âgés ; ces faits dessinent les contours d'un enjeu sociétal et économique majeur et expliquent la demande croissante en biomatériaux, implants à durée de vie accrue capables de combler les pertes osseuses.

Le recours aux greffes étant limité, et les matériaux d'origine animale pouvant poser des problèmes de biocompatibilité ou des risques d'infection, les efforts de recherche visent à élaborer des biomatériaux synthétiques capables de promouvoir la régénération osseuse.

On parle dans ce cas d'implants bioactifs : le matériau implanté n'est pas simplement destiné à combler de manière passive une perte osseuse en restant le plus inerte possible, mais au contraire il doit stimuler et participer activement au mécanisme de régénération osseuse. Ceci est particulièrement important dans le cas de larges défauts osseux, pour lesquels le mécanisme d'autoréparation ne fonctionne plus.

Actuellement les principaux matériaux bioactifs utilisés comme substituts osseux sont les « céramiques » bioactives, telles que les phosphates de calcium, et les verres bioactifs, également appelés « bioverres ».

Les premières céramiques bioactives ont été développées par L.L. Hench (L.L. Hench et al., J. Biomed. Mater. Res. 1971, 2, 117-141 ; L.L. Hench et al., J. Biomed. Mater. Res. 1973, 7, 25-42).

Les premiers verres bioactifs ont été préparés à partir de SiO₂, de P₂O₅, de CaO et de Na₂O. Les oxydes de silicium et de phosphore sont des formateurs de réseau qui participent à la cohésion du réseau vitreux. Les alcalins et alcalino terreux comme le sodium et le calcium ne présentent pas cette capacité et viennent modifier le réseau vitreux en y introduisant des ruptures de chaines qui sont à l'origine de la faible température de fusion de ces verres associée à un désordre structural accru. Leur présence a pour conséquence une plus grande réactivité des verres bioactifs à travers notamment leur corrosion dans un environnement aqueux. Cette réactivité permet la formation d'hydroxyapatite en milieu physiologique et favorise donc la reconstruction osseuse.

Le bioverre qui a été le plus étudié est un verre sodo-silico-phosphocalcique dit Bioglass® ou Bioverre de Hench. Sa composition de base est 45% SiO₂ - 24,5% CaO - 24,5% Na₂O - 6% P₂O₅, en masse par rapport à la masse totale de la composition. Les propriétés bioactives remarquables de ce matériau ne sont plus à démontrer. Le Bioglass® reste à l'heure actuelle un des matériaux bioactifs (induisant une réponse spécifique des cellules) les plus intéressants.

De nombreux développements ont été faits dans le domaine des verres bioactifs depuis leur découverte (M. Vallet-Regi et al., Eur. J. Inorg. Chem. 2003, 1029-1042), tels que l'incorporation de différents atomes ou l'incorporation de principes actifs. Les compositions des verres bioactifs ont été optimisées de façon à favoriser la prolifération des ostéoblastes et la formation de tissus osseux (WO 02/04606). L'incorporation d'argent a été proposée notamment pour conférer des propriétés antibactériennes aux verres bioactifs (WO 00/76486).

La demande WO 2009/027594 décrit, elle, un verre bioactif dans lequel le strontium est introduit en des quantités comprises entre 0,1 à 10% du poids total du verre bioactif.

Ces matériaux bioactifs présentent comme caractéristique d'être tout à la fois biocompatibles, capables de se lier spontanément aux tissus osseux, de promouvoir l'adhésion des cellules osseuses et, enfin d'être biorésorbables, étant progressivement remplacés par du tissu osseux néoformé à mesure que la repousse osseuse avance.

Pour le comblement de larges défauts osseux, les implants doivent avoir, en plus des caractéristiques précédentes, une morphologie spécifique : celle-ci s'inspire de l'os trabéculaire, à savoir une structure hautement poreuse constituée d'un réseau tridimensionnel de macropores interconnectés de plusieurs centaines de microns. En effet, dans le cas de larges défauts osseux, les cellules de l'os ont besoin d'une matrice "support" extracellulaire capable de guider et de stimuler l'adhésion, la prolifération, la différentiation cellulaire, tout en étant compatible avec les processus de vascularisation et d'invasion tissulaire.

Une telle structure macroporeuse est également requise pour les nouvelles applications envisagées en ingénierie tissulaire de l'os: il s'agit, à partir de cellules prélevées chez le patient, de fabriquer en laboratoire du tissu osseux nouveau que l'on pourra réimplanter a posteriori chez le patient. Pour être conduite de façon optimale, cette culture de tissu doit là aussi reposer sur des supports tridimensionnels poreux permettant une bonne adhésion cellulaire, la différentiation en cellules matures ainsi que la fabrication du tissu et en particulier la biominéralisation.

Joaquin Rodenas-Rochina et al. décrivent dans "Comparative study of PCL-HAp and PCL-bioglass composite scaffolds for bone tissue engineering" J. Mater Sci: Mater Med (2013) 24: 1293-1308, des implants en un matériau synthétique composite polymère bioverre ou polymère-hydroxyapatite ayant une telle structure macroporeuse.

Cependant, des implants possédant une structure mixte présentant à la fois une région dense et une région macroporeuse sont nécessaires en chirurgie maxillo-faciale et en orthopédie. Pour certaines applications, l'épaisseur requise pour la partie dense de l'implant peut être conséquente, jusqu'à plusieurs mm d'épaisseur. À l'heure actuelle, seule l'autogreffe ou l'allogreffe permettent de répondre à ce besoin. Il s'agit en effet des seules sources d'os mixtes possédant des parties corticales suffisamment volumineuses. Si l'autogreffe est le gold standard, le faible volume de prélèvement possible sur le patient lui-même et le risque de morbidité du site donneur sont des limitations sérieuses ; en outre, cela impose au patient une intervention chirurgicale supplémentaire. Concernant l'allogreffe, elle consiste ici en un prélèvement d'os massif sur donneurs cadavériques qui est complexe ; les quantités de greffons disponibles demeurent très limitées car les os massifs sont des tissus peu prélevés, leurs caractéristiques morphologiques doivent correspondre à celles du patient, et la réglementation concernant leur distribution est contraignante. Le développement de substituts synthétiques constitue une solution à ces problèmes.

En chirurgie dentaire, diverses techniques peuvent également nécessiter des implants à structure mixte poreuse/dense. Parmi elles, la technique de la régénération osseuse guidée fait appel à des barrières physiques pour empêcher la colonisation des tissus osseux par les tissus mous conjonctifs et épithéliaux, permettant ainsi seulement aux cellules à pouvoir ostéogénique d'envahir l'espace cicatriciel. À l'heure actuelle, des membranes synthétiques résorbables ou non résorbables sont utilisées pour jouer ce rôle de barrière, ou bien encore pour limiter la résorption d'un greffon osseux. Les membranes non résorbables type PTFE présentent l'inconvénient de devoir être fixées par des vis et d'une deuxième intervention pour les déposer, ainsi que les risques d'exposition et d'infection de la membrane. Les membranes résorbables sont de type collagénique ou synthétique et bien qu'elles diminuent les complications post-opératoires, elles nécessitent toujours un matériau de comblement soutenant la membrane : voir Hadi Antoun, Michel Karouni, Bouchra Sojos, La régénération osseuse guidée : résultats, limites et perspectives, Actualités Odonto-Stomatologiques 261 : 11-21, 2013.

En comparaison, un implant unique à structure mixte poreuse/dense présenterait l'avantage d'une véritable continuité entre la partie poreuse, à vocation de comblement, et la partie dense, jouant le rôle de barrière ; en plus d'être résorbable, des propriétés supplémentaires peuvent être conférées à la partie dense, comme par exemple la bioactivité.

En résumé, si de nombreux matériaux et formulations ont été développés pour le comblement des pertes osseuses, aucun ne répond totalement au cahier des charges décrivant l'implant idéal, à savoir :
- être biocompatible ;
- être bioactif : induire spontanément la formation d'un lien interfacial fort avec les tissus osseux, promouvoir l'adhésion et l'activité cellulaire ;
- être biorésorbable ;
- avoir une morphologie adéquate basée sur une matrice tridimensionnelle de macropores interconnectés, cette matrice tridimensionnelle de macropores interconnectés étant associée à une matrice tridimensionnelle n'ayant qu'un très faible nombre de pores ;
- avoir une bonne tenue mécanique ;
- être dérivé d'un procédé de fabrication permettant une mise en forme facile et suffisamment souple pour s'adapter à de nombreuses géométries de défauts et permettant d'obtenir deux parties : une partie dense et une partie poreuse.

Par morphologie adéquate basée sur une matrice tridimensionnelle de macropores interconnectés, on entend que la taille, la forme et la distribution des pores ainsi que la taille des interconnexions entre ces pores doivent être contrôlées.

Par association d'une matrice tridimensionnelle de macropores interconnectés et d'une matrice ayant un nombre faible de pores, on entend une matrice constituée, dans tous les cas, du même matériau hybride, et dont l'une des parties comprend un réseau tridimensionnel de macropores interconnectés et l'autre partie est dense.

Ainsi, l'invention a pour but de proposer un matériau qui répond parfaitement à tous ces critères et qui peut être fabriqué par des procédés qui permettent la réalisation d'architectures comprenant une association d'une partie poreuse et d'une partie dense composées d'une partie organique et d'une partie inorganique, sous forme de matériau hybride.

A cet effet, l'invention propose un matériau d'implant pour le comblement de défauts osseux, la régénération osseuse et l'ingénierie tissulaire de l'os, caractérisé en ce qu'il comprend une matrice en un matériau hybride comprenant :
- un polymère biodégradable P soluble dans au moins un solvant S1 et insoluble dans au moins un solvant S différent du solvant S1 et un verre bioactif à base de SiO₂ et CaO, contenant optionnellement P₂O₅ et/ou optionnellement dopé au strontium, caractérisé en ce qu'il comprend la superposition de :
- une partie poreuse ayant plus de 90% en nombre, de pores dont la plus grande dimension est supérieure ou égale à 100 µm, et
- une partie dense ayant plus de 80% en nombre, de pores dont la plus grande dimension est inférieure à 50 µm.

De préférence, le rapport volume partie dense/volume partie poreuse est compris entre 10/90 et 90/10.

Dans un premier mode de mise en œuvre, les pores de la partie poreuse ont tous la même forme et les mêmes dimensions à plus ou moins 10% près.

Par mêmes dimensions, on entend que les dimensions des pores ne varient pas de plus ou moins 10% les uns par rapport aux autres.

Dans un second mode de mise en œuvre, la partie poreuse a des pores dont la plus grande dimension décroit de la base de l'implant vers la partie dense.

Dans un troisième mode de mise en œuvre, la partie poreuse a des pores dont la plus grande dimension croit de la base de l'implant vers la partie dense.

Dans un premier mode de réalisation, la partie poreuse et la partie dense sont en superposition planaire.

Dans un second mode de réalisation, la partie poreuse et la partie dense sont en superposition concentrique.

Les pores de la partie poreuse peuvent avoir une forme sphérique ou de polygones, de préférence la forme de carrés.

L'invention propose également un procédé de fabrication d'un matériau d'implant selon l'invention ayant des pores sphériques, caractérisé en ce qu'il comprend les étapes suivantes :
a) sélection des précurseurs alcoxydes d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S différent du solvant S1,
c) sélection de microsphères d'un agent porogène A ayant des diamètres et des tailles correspondant aux diamètres et tailles recherchés des pores dans le matériau constituant l'implant à fabriquer, cet agent porogène A étant :
   - en un polymère insoluble dans le au moins un solvant S1 et soluble dans le au moins un solvant S,
   le au moins un solvant S dans lequel le matériau du polymère biodégradable P est insoluble et le au moins un solvant S dans lequel le matériau de l'agent porogène A est soluble étant identiques,
d) introduction des microsphères de l'agent porogène A dans un moule ayant la forme et la taille recherchées pour l'implant, ces microsphères formant un empilement compact correspondant à la taille et à la forme des pores à obtenir pour la partie poreuse du matériau d'implant, et représentant entre 5% et 50% en volume, par rapport au volume total du mélange agent porogène A-polymère biodégradable P-précurseurs alcoxydes du verre bioactif M,
e) introduction du polymère biodégradable P dans les précurseurs alcoxydes du verre bioactif M,
f) introduction du mélange obtenu à l'étape e) dans le moule,
g) gélification du mélange contenu dans le moule après l'étape f),
h) démoulage du mélange obtenu à l'étape g),
i) élimination des microsphères d'agent porogène A par lavage avec le solvant S.

L'invention propose encore un procédé de fabrication d'un matériau d'implant selon l'invention dans lequel les pores peuvent avoir toute forme voulue, y compris celle de polygones, caractérisé en ce qu'il comprend les étapes suivantes :
a) sélection des précurseurs alcoxydes d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S différent du solvant S1,
c) fabrication, par impression 3D, d'une préforme, en un polymère insoluble dans le au moins un solvant S1 et soluble dans le au moins un solvant S, cette préforme étant la réplique inverse en terme de forme et de taille finales voulues pour les pores dans la partie poreuse de l'implant final et représentant entre 5 et 50% en volume du volume total du mélange préforme-polymère biodégradable P-précurseur alcoxydes du verre bioactif M,
d) introduction de la préforme dans un moule ayant la forme et la taille recherchées de l'implant final,
e) introduction du polymère biodégradable P dans les précurseurs alcoxydes du verre bioactif M,
f) introduction du mélange obtenu à l'étape e) dans le moule,
g) gélification du mélange contenu dans le moule après l'étape f),
h) démoulage du mélange obtenu à l'étape g),
i) élimination de la préforme par lavage avec le solvant S.

Dans ces deux procédés les étapes e) et/ou f) peuvent être mises en œuvre avant l'étape d).

Mais, les étapes d), e) et f) peuvent également être mises en œuvre simultanément.

Pour obtenir un matériau d'implant dans lequel la partie poreuse et la partie dense du matériau d'implant sont en superposition planaire, à l'étape d), l'empilement compact de microsphères ou la préforme sont placés de façon à toucher les parois latérales du moule, en laissant un espace libre au dessus de l'empilement de microsphères ou de la préforme.

Pour obtenir un matériau d'implant dans lequel la partie poreuse et la partie dense du matériau d'implant sont en superposition concentrique, à l'étape d), l'empilement compact de microsphères ou la préforme sont placés au centre du moule en laissant un espace libre entre l'empilement compact de microsphères ou les parois latérales de la préforme et les parois latérales du moule.

De préférence, le polymère biodégradable P est un polymère biodégradable soluble dans au moins un solvant S1 et insoluble dans au moins un solvant S, choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone),
- les protéines, de préférence la gélatine ou le collagène,
et le matériau de l'agent porogène ou de la préforme est un matériau choisi parmi les polymères biodégradables insolubles dans le au moins un solvant S1 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène, ou l'acrylonitrile butadiène styrène (ABS),
le matériau de l'agent porogène A ou de la préforme étant différent du polymère biodégradable P.

Egalement de préférence, le rapport en poids polymère biodégradable P/ verre bioactif M est compris entre 20/80 et 80/20, bornes incluses.

Toujours de préférence, le verre bioactif M est un verre à base de SiO₂ et de CaO, le polymère biodégradable P est la gélatine, le matériau de la préforme est l'ABS et le solvant S est l'acétone.

Lorsque l'empilement des microsphères est utilisé pour créer les pores, de préférence le verre bioactif M est un verre à base de SiO₂ et de CaO, le polymère biodégradable P est la gélatine, le matériau de l'agent porogène A est le polyméthacrylate de méthyle et le solvant S est l'acétone.

Les procédés de l'invention peuvent, de plus, comprendre, à l'étape f), une étape d'introduction d'un agent de couplage, de préférence un composé organoalkoxysilane, plus préférablement du 3-glycidoxypropyltriméthoxysilane (GPTMS), encore plus préférablement du 3-glycidoxypropyltriéthoxysilane (GPTES).

L'invention propose enfin un implant en un matériau hybride pour le comblement de défauts osseux, la régénération osseuse et l'ingénierie tissulaire de l'os, caractérisé en ce qu'il comprend un matériau d'implant selon l'invention ou obtenu par l'un ou l'autre des procédés de l'invention.

L'invention sera mieux comprise et d'autres caractéristiques et avantages de celle-ci apparaîtront plus clairement à la lecture de la description explicative qui suit et qui est faite en référence aux figures annexées dans lesquelles :
- la figure 1 est une représentation schématique d'un implant selon l'invention, dans lequel la partie poreuse a des pores ayant tous la même dimension et la partie dense est superposée en une structure planaire sur la partie poreuse,
- la figure 2 est une représentation schématique d'un implant selon l'invention, dont les pores de la partie poreuse présentent un gradient de dimension de pores décroissants de la base de l'implant vers la partie dense de l'implant qui est superposée en une configuration planaire sur la partie poreuse de l'implant,
- la figure 3a est une photographie d'un implant selon l'invention dans lequel la partie poreuse et la partie dense forment une structure en superposition planaire,
- la figure 3b est une photographie d'une coupe de l'implant montré en figure 3a mais vu de côté selon l'invention,
- la figure 3c représente une photographie prise au microscope électronique à un grandissement de x30 de l'implant représenté en figure 3b, à l'interface de la partie poreuse et de la partie dense,
- la figure 4 représente un organigramme du premier procédé de fabrication d'un implant en un matériau hybride (à base de verre bioactif et de gélatine) selon l'invention,
- la figure 5a représente schématiquement une préforme, obtenue par impression 3D, utilisée dans le second procédé de fabrication d'un implant en un matériau hybride selon l'invention,
- la figure 5b est une photographie d'une coupe de l'implant selon l'invention, dans lequel la partie poreuse et la partie dense sont superposées en une configuration planaire,
- la figure 5c montre une photographie prise au microscope électronique à balayage à un grandissement de x30 de l'implant montré en figure 5b à l'interface partie poreuse/partie dense,

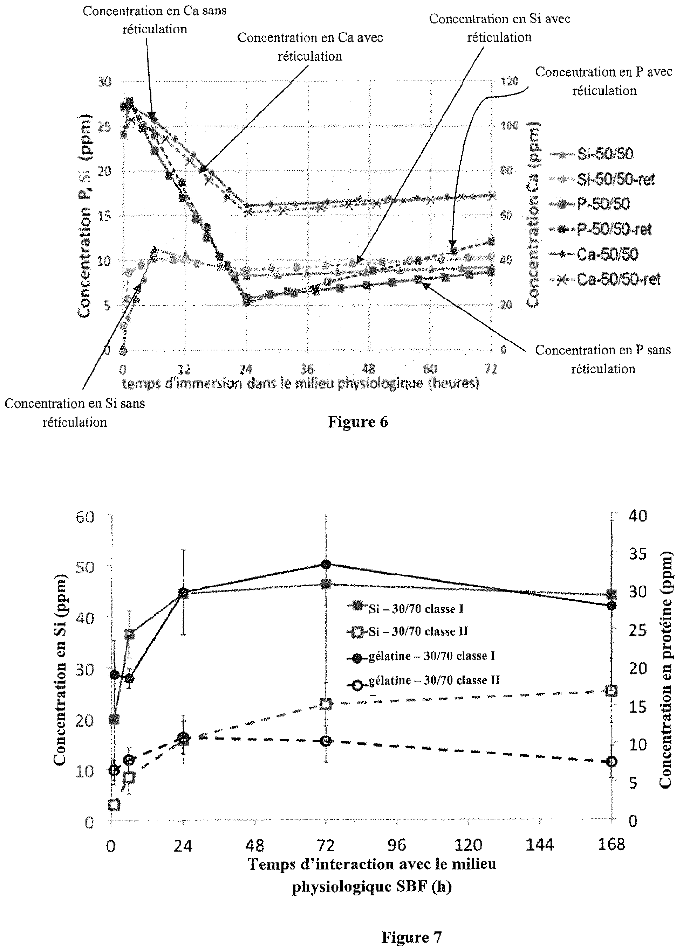

- la figure 6 montre les courbes d'évolution en fonction du temps de la composition (en ppm) du milieu physiologique mis au contact d'implants hybrides de classe I en verre bioactif/gélatine (50%(SiO₂-CaO)/50%gélatine) selon l'invention,
- la figure 7 montre la courbe d'évolution en fonction du temps de la concentration (en ppm) en silicium et protéines du milieu physiologique (SBF) mis au contact d'implants hybrides de classe I et de classe II en verre bioactif/gélatine (30%(SiO₂-CaO)/70%gélatine) selon l'invention,
- la figure 8 montre les courbes d'évolution en fonction du temps de la concentration en calcium et phosphore (en ppm) du milieu physiologique (SBF) mis au contact d'implants hybrides de classe I et de classe II en verre bioactif/gélatine (30%(SiO₂-CaO)/70%gélatine) selon l'invention,
- la figure 9a montre une photographie d'une coupe d'un implant en un matériau hybride de classe I constitué de 30% de bioverre/70% gélatine B dont une partie est dense et l'autre partie est poreuse en superposition planaire, obtenu à l'exemple 1,
- la figure 9b montre une photographie prise au microscope électronique à balayage à un grandissement de x20 d'une coupe de l'implant hybride montré en figure 9a,
- la figure 10a montre une photographie d'une coupe d'un implant en un matériau hybride de classe I constitué de 30% de bioverre/ 70% gélatine B dont une partie est dense et l'autre partie est poreuse en superposition concentrique, obtenu à l'exemple 2,
- la figure 10b montre une vue prise au microscope électronique à balayage à un grandissement de x20 d'une coupe de l'implant en un matériau hybride montré en figure 10a,
- la figure 11a montre une photographie d'une coupe d'un implant en un matériau hybride de classe II constitué de 30% de bioverre/ 70% de gélatine B dont une partie est dense et l'autre partie est poreuse en superposition planaire, obtenu à l'exemple 3,
- la figure 11b montre une photographie prise au microscope électronique à balayage à un grandissement de x20 d'une coupe de l'implant montré en figure 11a,
- la figure 12a montre une photographie d'une coupe d'un implant en un matériau hybride de classe I constitué de 30% de bioverre/ 70% d'acide poly-DL-lactique (PDLLA) dont une partie est dense et l'autre partie est poreuse en superposition planaire, obtenu à l'exemple 4,
- la figure 12b montre une photographie prise au microscope électronique à balayage à un grandissement de x20 d'une coupe de l'implant montré en figure 12a,
- la figure 13a montre une photographie d'une coupe d'un implant en un matériau hybride de classe II constitué de 30% de bioverre/ 70% de polycaprolactone (PCL) dont une partie est dense et l'autre partie est poreuse en superposition planaire, obtenu à l'exemple 5,
- la figure 13b montre une photographie prise au microscope électronique à balayage à un grandissement de x20 d'une coupe de l'implant en un matériau hybride montré en figure 13a,
- la figure 14a montre une photographie d'une coupe d'un implant en un matériau hybride de classe I constitué de 30% de bioverre/ 70% de PDLLA avec une porosité dite aléatoire, obtenu à l'exemple 6,
- la figure 14b montre une photographie prise au microscope électronique à balayage à un grandissement de x20 d'une coupe de la partie poreuse de l'implant en un matériau hybride montré en figure 14a,
- la figure 15a montre une photographie d'une coupe d'un implant en un matériau hybride de classe II constitué de 30% de bioverre/ 70% de PCL avec une porosité dite aléatoire, obtenu à l'exemple 7,
- la figure 15b montre une photographie prise au microscope électronique à balayage à un grandissement de x20 d'une coupe de la partie poreuse de l'implant en un matériau hybride montré en figure 15a,
- la figure 16a montre une photographie d'une coupe d'un implant en un matériau hybride de classe I constitué de 30% de bioverre/ 70% de PCL avec une taille de pores croissant de la base de l'implant vers le haut de l'implant, obtenu à l'exemple 9,
- la figure 16b montre une photographie prise au microscope électronique à balayage à un grandissement de x20 d'une coupe de l'implant en un matériau hybride montré en figure 16a,
- la figure 17a montre une photographie de l'implant obtenu à l'exemple 10,
- la figure 17b montre une photographie prise au microscope électronique à balayage à un grandissement de x50 d'une coupe de l'implant obtenu à l'exemple 10, à l'interface dense/poreux,
- la figure 18a montre une photographie prise au microscope électronique à balayage à un grandissement de x50 d'une coupe de la préforme utilisée lors de la fabrication du matériau d'implant obtenu à l'exemple 11,
- la figure 18b montre une photographie prise au microscope électronique à balayage à un grandissement de x70 d'une coupe de l'implant en un matériau hybride obtenu à l'exemple 11, à l'interface dense/poreux,
- la figure 19a montre une photographie prise au microscope électronique à balayage à un grandissement de x30 d'une coupe de la préforme utilisée lors de la fabrication du matériau d'implant obtenu à l'exemple 12,
   la figure 19b montre une photographie prise au microscope électronique à balayage à un grandissement de x70 d'une coupe de l'implant en un matériau hybride obtenu à l'exemple 12, à l'interface dense/poreux,
- la figure 20a montre une photographie du matériau d'implant obtenu à l'exemple 13,
- la figure 20b montre une photographie prise au microscope électronique à balayage à un grandissement de x50 d'une coupe de l'implant en un matériau hybride obtenu à l'exemple 13, à l'interface dense/poreux,
- la figure 21a montre une photographie du matériau d'implant obtenu à l'exemple 14, et
- la figure 21b montre une photographie prise au microscope électronique à balayage à un grandissement de x50 d'une coupe de l'implant en un matériau hybride obtenu à l'exemple 14, à l'interface dense/poreux,

Dans ce qui précède et ce qui suit, les termes suivants ont les significations suivantes :
- "partie poreuse" : partie du matériau d'implant dans laquelle plus de 90% en nombre des pores ont leur plus grande dimension supérieure ou égale à 100 µm,
- "partie dense" : partie du matériau d'implant dans laquelle plus de 80% en nombre des pores ont leur plus grande dimension inférieure à 50 microns,
- "interconnexion(s) entre pores" : ouverture(s) permettant le passage d'un pore à l'autre,
- "milieu aqueux" : tout milieu liquide contenant de l'eau, ou eau seule,
- "biodégradable" : dégradable dans un liquide physiologique, par exemple une solution saline tamponnée (SBF),
- "biorésorbable" : éliminable dans un milieu physiologique contenant des cellules biologiques,
- "pore sphérique" ou "sphère" : pore ou sphère dont le rapport du plus petit diamètre sur le plus grand diamètre est de 0,9 ± 0,1,
- "empilement compact de microsphères d'agent porogène A" : empilement de microsphères d'agent porogène A dans lequel :
   au moins 70% en nombre, de préférence plus de 95% en nombre de microsphères sont en contact les unes avec les autres, et restent en contact les unes avec les autres lorsque le mélange agent porogène A et hybride polymère biodégradable P-verre bioactif M-sont dans le moule, et lorsque l'empilement de microsphères est recouvert et infiltré avec le mélange hybride verre bioactif M-polymère biodégradable P.

On peut obtenir un tel empilement compact de microsphères d'agent porogène A par centrifugation du mélange microsphères d'agent porogène A et hybride polymère biodégradable P-verre bioactif M ou encore en appliquant une pression négative (vide) ou positive (supérieure à la pression atmosphérique) sur le mélange microsphères d'agent porogène A et hybride polymère biodégradable P-verre bioactif M introduit dans le moule, avant et pendant la gélification de ce mélange.

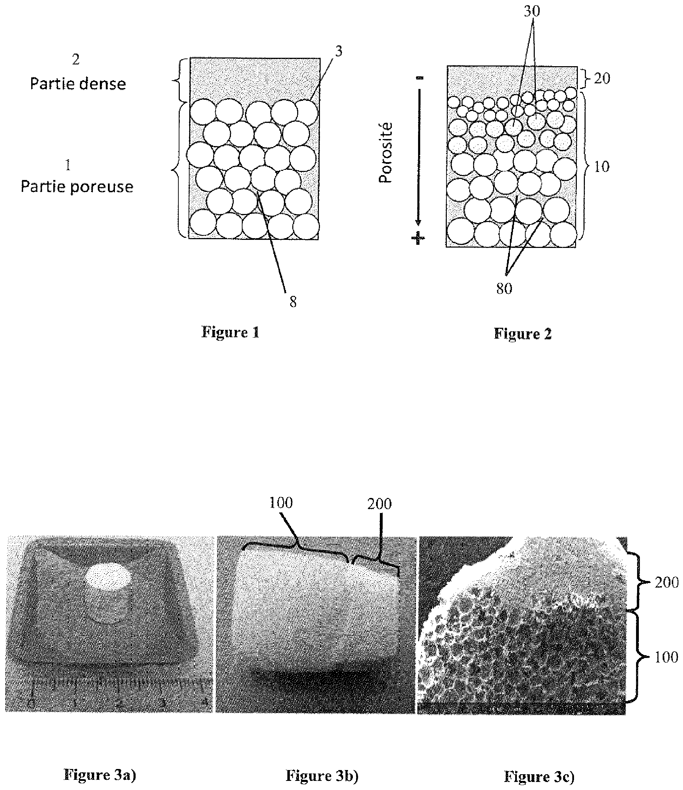

Le matériau d'implant pour le comblement de défauts osseux, la régénération osseuse et l'ingénierie tissulaire de l'os sera décrit en relation avec les figures 1, 2, 3a) - 3c), 5b) et 5c).

Comme on le voit sur ces figures, le matériau d'implant de l'invention comprend une matrice comprenant une partie poreuse, notée 1, 10, 100, 1000, 10000 dans les figures, et une partie dense, notée 2, 20, 200, 2000, 20000 dans les figures, dite dense, c'est-à-dire dans laquelle les pores ont une taille inférieure à 50 microns.

La partie poreuse et la partie dense sont superposées, l'une sur l'autre, ou l'une autour de l'autre, sans aucune matière ou couche ajoutée. Le matériau forme une seule pièce.

La matrice du matériau d'implant de l'invention est constituée d'une phase organique et d'une phase inorganique.

La phase inorganique est un verre bioactif M.

Les céramiques bioactives et les verres bioactifs sont bien connus de l'homme du métier et sont décrits en particulier dans L.L. Hench et al., J. Biomed. Mater. Res. 1971, 2, 117-141 ; L.L. Hench et al., J. Biomed. Mater. Res. 1973, 7, 25-42 pour les céramiques bioactives et dans M. Vallet-Regi et al., Eur. J. Inorg. Chem. 2003, 1029-1042 et WO 02/04606, WO 00/76486 et WO 2009/027594, en particulier. Dans l'invention, on utilise uniquement un verre bioactif.

La partie organique du matériau d'implant de l'invention est un polymère biodégradable P soluble dans au moins un solvant S1 et insoluble dans au moins un solvant S. Ces solvants peuvent être de l'eau, un milieu aqueux ou encore un solvant organique. De préférence, le polymère biodégradable P est choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone), et
- les protéines, de préférence la gélatine ou le collagène.

La matrice du matériau d'implant de l'invention est constituée du verre bioactif M et du polymère biodégradable P qui forment un matériau hybride, c'est-à-dire formant une seule phase.

Le matériau hybride utilisé dans l'invention est obtenu par un procédé qui comprend la formation d'un sol contenant tous les précurseurs alcoxydes du verre bioactif, l'ajout du polymère biodégradable P dans ce sol et la gélification de la solution ainsi obtenue par une succession de réactions de polymérisation (polymérisation sol-gel de la phase inorganique) (condensation des alcoxydes). On obtient alors un mélange hybride associant intimement la phase minérale et la phase organique.

La partie poreuse 1, 10, 100, 1000, 10000 de l'implant de l'invention peut avoir des pores 3 ayant tous la même taille, c'est-à-dire des pores dont les dimensions ne présentent pas une variation de plus ou moins 10% les uns par rapport aux autres comme montré en figure 1 ou avoir des tailles de pores 30 décroissant du bas de la partie poreuse jusqu'au haut de la partie poreuse, comme montré en figure 2, ou inversement,
Mais, la taille des pores peut également être alternée (une rangée de pores de diamètre plus grand que la rangée suivante etc...).

Sur, ou autour, de cette partie 1, 10, 100, 1000, 10000 poreuse, est superposée en liaison directe une partie dense 2, 20, 200, 2000, 20000 constituée du même matériau hybride que la partie poreuse 1, 10, 100, 1000, 10000 mais cette fois-ci sans porosité créée.

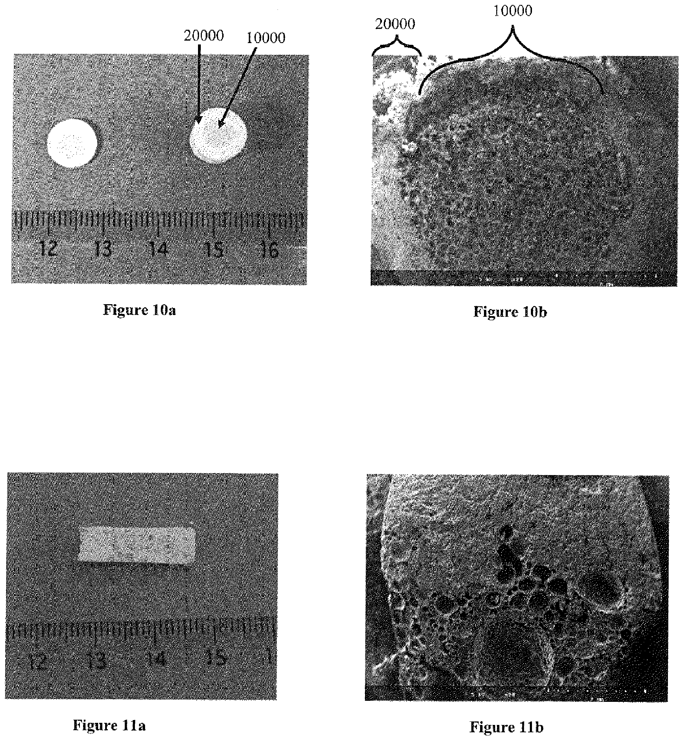

Aussi, comme on le voit en figures 1, 2, 3a, 3b, 3c et 5b et 5c, la partie 2, 20, 200, 2000 peut être superposée en une configuration planaire au dessus de la partie poreuse 1, 10, 100, 1000. Elle peut également être, comme montré en figures 10a et 10b, où elle est notée 20000, placée autour de la partie poreuse notée 10000, formant une configuration concentrique.

Un premier procédé de fabrication de l'implant de l'invention est un procédé faisant appel à un agent porogène A qui est constitué de microsphères en un polymère soluble dans au moins un solvant S dans lequel le polymère biodégradable P n'est, lui, pas soluble.

Ainsi, le procédé de l'invention consiste à empiler des microsphères d'agent porogène A en un matériau polymère, différent du polymère biodégradable P, dans un moule ayant la forme et la taille correspondant à la géométrie du défaut osseux à combler ou du défaut où la régénération osseuse est voulue.

Ces microsphères d'agent porogène A permettent d'obtenir au final des pores dont la taille et la distribution correspondront en négatif à l'empilement de microsphères d'agent porogène A initialement réalisé.

Cet empilement de microsphères d'agents porogènes permet d'obtenir la partie poreuse 1, 10, 100, 10000 du matériau d'implant.

En effet, le matériau destiné à constituer la matrice du matériau d'implant de l'invention sera ensuite infiltré dans l'empilement des billes de microsphères d'agents porogènes A et, également, au dessus, ou autour, de cet empilement, puis ensuite solidifié pour pouvoir être démoulé sans changer la forme et la taille de l'empilement de l'implant voulu. L'agent porogène A sera alors éliminé permettant l'obtention du matériau d'implant de l'invention formé de la superposition d'une partie à porosité contrôlée, et d'une partie dense dans laquelle les pores ont leur plus grande dimension inférieure à 50 micromètres.

Comme on le voit, ce procédé n'utilise aucun traitement thermique à haute température pour fritter le verre bioactif M, la seule température nécessaire étant la température d'évaporation du solvant S utilisé.

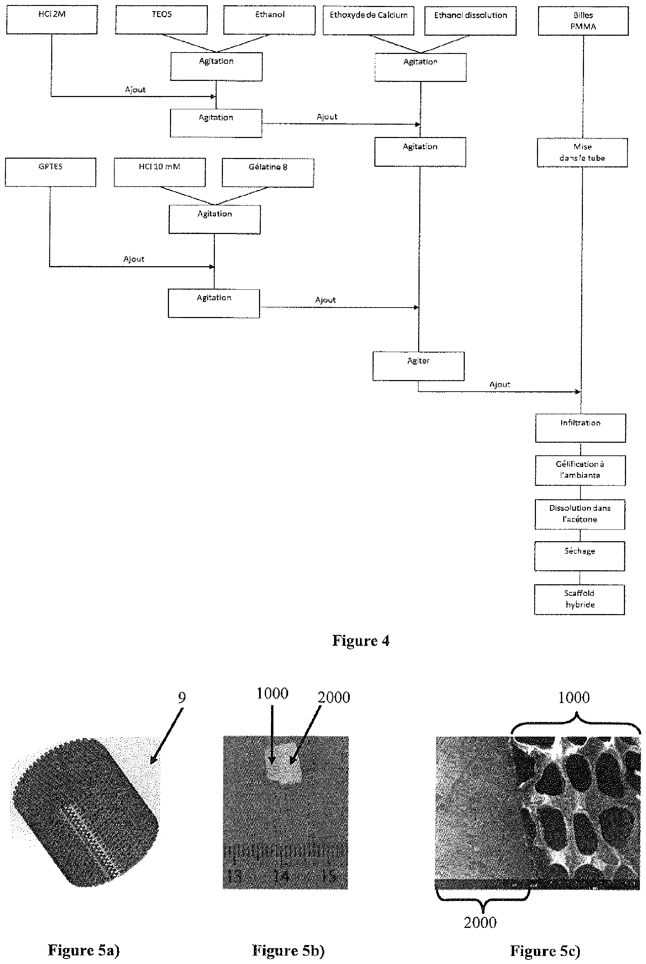

La figure 4 montre un organigramme de ce procédé de fabrication.

Comme on le voit en figure 4, dans une première étape l'agent porogène est placé dans un récipient de forme adéquate, correspondant à la géométrie du défaut osseux à combler. L'agent porogène est placé en un empilement de sphères en un matériau polymére. L'agent porogène, doit impérativement pouvoir être éliminé sans traitement thermique afin de préserver la partie organique du matériau hybride à élaborer. Il sera donc choisi parmi la liste suivante :
- les polyméthacrylates d'alkyle en C₁ à C₄, par exemple : le polyméthacrylate de méthyle ou le polyméthacrylate de butyle,
- le polyuréthane,
- l'acide polyglycolique,
- les différentes formes d'acides polylactiques,
- les copolymères d'acides lactique-coglycoliques,
- le polycaprolactone,
- le polypropylène fumarate,
- la paraffine et le naphtalène,
- l'acrylonitrile butadiène styrène (ABS).

On préfèrera utiliser des microsphères de PMMA comme agent porogène. Un des avantages du PMMA est qu'il peut être aisément dissous par de nombreux solvants. D'autre part, dans le cas où des résidus de PMMA non éliminés viendraient à subsister, la bonne biocompatibilité du PMMA avec les tissus humains est une garantie que l'implant ne présentera aucun risque de cytotoxicité.

Afin de contrôler la porosité, et éventuellement de l'organiser, il est avantageux d'utiliser des particules sphériques, à savoir des billes de PMMA. Leur diamètre peut être choisi entre une centaine à plusieurs centaines de microns, suivant les applications.

La porosité du matériau qui sera finalement obtenu peut être contrôlée suivant trois points.

Premièrement le diamètre des pores qui seront obtenus dépend directement du diamètre des particules porogènes initiales. Il suffit donc d'ajuster la granulométrie des billes PMMA initiales en vue d'obtenir très simplement la porosité désirée.

Deuxièmement la taille des interconnexions entre pores dépend directement de la taille de la zone de contact entre les billes polymères dans l'empilement initial. La taille de cette zone de contact peut être modifiée en faisant fusionner entre elles les particules de polymère initiales, au moyen d'un solvant, ou par un traitement thermique préliminaire.

Troisièmement, l'organisation initiale des billes de porogène dont la plus grande dimension est comprise entre 100µm et 900µm, se fera soit de manière aléatoire, soit de manière stratifiée par taille de billes, mais toujours de manière à avoir un empilement compact de billes laissant suffisamment de place dans le moule pour permettre de remplir avec un surplus de matériau hybride sous forme de sol afin d'obtenir une partie dense pour l'implant.

Cette organisation initiale des billes pourra également être un empilement compact de billes en agent porogène A, ces billes ayant toutes la même forme et les mêmes dimensions. Par surplus de sol hybride, on entend que la quantité de sol hybride introduite dans le moule doit être supérieure au volume laissé vacant par les billes d'agent porogène (y compris l'espace interstitiel accessible entre ces billes).

Lorsque ce surplus est placé autour de l'empilement de billes, auquel cas l'empilement de billes est placé au centre du moule pour permettre au surplus hybride destiné à former la partie dense de se placer entre les parois latérales du moule et les parois latérales de l'empilement de billes, on obtient une superposition concentrique de la partie poreuse et de la partie dense du matériau d'implant. Lorsque ce surplus est placé au dessus de la partie poreuse, auquel cas l'empilement de billes sera placé au fond du moule avec ses parois latérales en contact avec les parois latérales du moule, en laissant la place pour le surplus de sol hybride au dessus de l'empilement de billes, on obtient un matériau d'implant dans lequel la partie poreuse et la partie dense sont en superposition planaire.

Dans une deuxième étape, une trame organique/inorganique composée d'une phase en polymère biodégradable et d'une phase inorganique bioactive infiltre la structure porogène en polymère ; le polymère intervenant dans la matrice hybride, et qui est mêlé au verre bioactif doit présenter toutes les caractéristiques de biocompatibilité, biorésorbabilité, et pouvoir être aisément mis en forme sans l'intervention de produits pouvant laisser des résidus cytotoxiques. Ce polymère sera choisi parmi la liste suivante :
- les polysaccharides biorésorbables, par exemple : le dextran, l'acide hyaluronique, l'agar, le chitosane, l'acide alginique, l'alginate de sodium ou de potassium, le galactomannane, la carraghénane, la pectine,
- les polyesters biorésorbables, par exemple : l'alcool polyvinylique (PVA) ou le poly(acide lactique) (PLA),
- les polymères synthétiques biodégradables, par exemple : le polyéthylène glycol (PEG) ou le poly(caprolactone) (PCL),
- les protéines, par exemple : la gélatine ou le collagène.

Dans un mode de réalisation de l'invention, le choix du bio-polymère s'est porté sur la gélatine. En effet la gélatine est un bio-polymère naturel, biodégradable, biocompatible, bon marché et facilement disponible. La gélatine est qui plus est dérivée du collagène naturellement présent dans les os. Elle est d'autre part déjà utilisée dans le cadre d'applications cliniques (pansements, adhésifs, encapsulation de substances pharmaceutiques).

La partie inorganique de la matrice hybride consiste quant à elle en un verre bioactif, à cause i) de leur grande capacité à induire la minéralisation, ii) la possibilité de façonner leurs propriétés texturales et morphologiques (porosité, taille, et donc surface spécifique) à l'échelle nanométrique, iii) la grande gamme de compositions bioactives qu'il est possible de formuler, en y ajoutant par exemple des éléments anti-inflammatoires, ou ostéoinducteurs, iv) enfin la combinaison de leurs propriétés de bioactivité et de biorésorbabilité qui en font les biomatériaux les plus prometteurs pour la régénération osseuse, notamment par rapport aux phosphates de calcium, qui sont généralement soit moins bioactifs, soit moins résorbables.

Suivant l'invention, la trame organique/inorganique est réalisée de manière à obtenir une matrice hybride. La matrice hybride est obtenue en intégrant le polymère en amont, dès le processus de synthèse des verres bioactifs, qui est basé sur le procédé sol-gel. Brièvement, au cours du procédé sol-gel une solution contenant tous les précurseurs chimiques du verre bioactif est amenée à gélifier par une succession de réactions de polymérisation ; dans notre cas, le polymère biocompatible (par exemple la gélatine) est ajouté avant gélification du sol, de manière à obtenir un mélange hybride associant intimement les espèces minérales et organiques. Le mélange hybride se distingue donc d'un mélange composite par une intrication intime entre les deux phases organiques et inorganiques, ces deux phases étant indiscernables (sauf à l'échelle moléculaire) dans le cas d'un mélange hybride. Cela est typiquement le cas lorsque les domaines de taille des phases organique et inorganique sont inférieurs à quelques dizaines de nanomètres. Pour la réalisation d'hybrides, une difficulté majeure est que les traitements thermiques à haute et moyenne température (> 150°C) sont à proscrire ; or dans les processus habituelles ces traitements thermiques sont indispensables à l'obtention d'un réseau vitreux homogène. L'invention décrit ici une nouvelle voie de synthèse menée à température modérée (≤ 60°C), proche de l'ambiante. Notamment l'utilisation d'un précurseur alcoxyde pour le calcium permet l'incorporation de cette espèce dans la phase inorganique sans traitement thermique. Ce procédé spécifique est décrit en figure 4, en utilisant dans cet exemple un empilement de billes PMMA comme architecture porogène. Une fois formé et avant gélification totale, le mélange hybride est versé sur la structure porogène. De plus, il peut être avantageux d'ajouter un agent couplant au mélange, tel qu'un organo-alcoxysilane ; par exemple, celui-ci peut être simplement ajouté au polymère biocompatible préalablement dissous. Le rôle de l'agent couplant est de fonctionnaliser le polymère biocompatible, en vue de permettre l'établissement de liaisons covalentes avec la phase inorganique (réseau silicate du verre bioactif). Un véritable copolymère organo-minéral est ainsi obtenu. L'intérêt est de pouvoir maîtriser à façon la dégradabilité de l'implant hybride ainsi que sa tenue mécanique, en agissant simplement sur le nombre de liaisons chimiques créées entre les phases organique et inorganique, ce nombre de liaisons chimiques créées dépendant de la quantité d'agent couplant introduite. Un exemple d'agent couplant utilisé avec succès par les inventeurs est le GPTMS (3-glycidoxypropyltrimethoxysilane), qui est soluble par exemple dans une solution aqueuse de gélatine.

Dans une troisième étape, le contenant et son mélange subissent une opération de gélification pendant plusieurs heures assurant la « prise » du mélange. Cette opération peut être menée à température modérée proche de l'ambiante (≤60°C) afin de ne pas dégrader la partie organique de l'hybride.

Dans une quatrième étape, l'architecture polymère en matériau porogène est éliminée par dissolution dans un solvant approprié.

Dans les exemples décrits en figure 4, les billes PMMA sont éliminées par lavage à l'acétone. Le choix de l'acétone présente plusieurs intérêts. Tout d'abord les billes de PMMA sont facilement dissoutes dans l'acétone ; la gélatine est quant à elle insoluble dans l'acétone ; l'acétone permet de plus de poursuivre si nécessaire la déshydratation de la gélatine ; enfin c'est un solvant d'usage très courant, relativement économique, particulièrement disponible, recyclable, qui ne présente pas de risque sérieux de toxicité.

Après plusieurs étapes de lavage, l'empreinte porogène initiale est complètement éliminée et le matériau hybride final en verre bioactif/polymère biocompatible est obtenu sous la forme d'un bloc, macro-poreux en totalité ou en partie. Sa dégradabilité en milieu vivant et sa tenue mécanique peuvent en sus être ajustées facilement en réticulant le polymère biocompatible lors d'une ultime étape d'immersion dans une solution d'un agent réticulant comme par exemple la génipine, le carbodiimide, le glutaraldéhyde, le formaldéhyde.

Les structures obtenues peuvent être lavées sans aucun dommage dans des bains d'éthanol, afin d'éliminer d'éventuels résidus indésirables (chlorure, acétone, etc.).

Un second procédé pour fabriquer l'implant selon l'invention est basé sur l'emploi d'une préforme, en un matériau polymère, réalisée par impression 3D, en tant qu'agent porogène. L'avantage de ce procédé est de pouvoir ajuster la porosité (formes et tailles des pores) à façon.

Comme montré en figure 5a, une préforme en un matériau polymère choisi parmi les mêmes matériaux que ceux cités précédemment comme matériaux de l'agent porogène A, plus préférablement une préforme en acrylonitrile butadiène styrène (ABS) à maille carrée est utilisée à la place des billes de PMMA, dans l'organigramme montré en figure 4.

Ainsi, après l'élimination de la préforme (9), la réplique inverse de cette préforme est aisément obtenue, en l'occurrence une matrice hybride hautement poreuse avec des pores de taille parfaitement contrôlée périodiquement espacés, et des murs de taille régulière. De la même manière que précédemment, une partie dense est obtenue dans l'implant lorsque le récipient est rempli non seulement au niveau de la préforme (9) mais également au-dessus de cette préforme. On obtient alors un matériau d'implant tel que montré en figure 5b ayant une structure planaire.

Le récipient peut également être rempli non seulement au niveau de la préforme (9) mais avec la préforme ne touchant pas les parois latérales du récipient et l'espace entre les parois latérales du récipient étant rempli de matériau hybride seul, sans agent porogène.

On obtient alors un matériau d'implant ayant une structure concentrique.

La préforme lorsque en ABS, peut être éliminée par lavage dans de l'acétone.

On comprendra immédiatement que le matériau des microsphères de l'agent porogène A et celui de la préforme (9) doivent être différents du polymère biodégradable P utilisé pour obtenir le matériau hybride de l'implant de l'invention.

Egalement, la préforme (9) peut constituer elle-même le récipient.

Afin de mieux faire comprendre l'invention, on va maintenant décrire à titre d'exemples purement illustratifs et non limitatifs plusieurs modes de réalisation.

### Exemple 1 :

Fabrication d'un matériau d'implant selon l'invention avec une matrice en matériau hybride avec une partie dense et une partie poreuse en superposition planaire.

On a débuté par l'étape d'empilement compact des microsphères d'agent porogène en polyméthacrylate de méthyle dans un moule ayant la géométrie recherchée pour l'implant et avec un volume supérieur à la taille de l'implant. L'empilement compact de billes d'agent porogène représentait 40% en volume, par rapport au volume total du mélange agent porogène A-polymère biodégradable P-précurseurs alcoxydes du verre bioactif M. Le matériau de l'agent porogène A était le polyméthacrylate de méthyle. Les sphères avaient un diamètre compris entre 400 et 600 µm.

Dans une deuxième étape, le mélange hybride a été versé dans le moule contenant l'empilement de billes. Le volume de mélange hybride est strictement supérieur au volume laissé par les interstices entre les billes. Le volume est ajusté pour remplir tout le volume utile du moule. Ainsi, le volume de sol hybride est égal à la différence entre le volume utile du moule et le volume occupé par les billes.

Une centrifugation ou une infiltration sous pression ou une infiltration sous vide peuvent être utilisées pour aider le mélange hybride à remplir les interstices entre les microsphères de poly(méthacrylate de méthyle). Un bouchon, dont la surface est plane, est placé au contact du sol avec l'aide d'une légère pression dans le but d'obtenir une surface plane sur l'implant.

Le matériau hybride a été obtenu par un procédé sol-gel.

Dans ce procédé, un sol contenant tous les précurseurs alcoxydes du verre bioactif est amené à gélifier par une succession de réactions de polymérisation.

Les précurseurs alcoxydes étaient en des quantités telles que la composition du verre bioactif était 75% SiO₂ et 25% CaO, en masse, par rapport à la masse totale du verre bioactif obtenu au final.

Dans le cas du présent exemple, la gélatine (le polymère biodégradable P) a été ajoutée avant gélification du sol, de manière à obtenir un mélange hybride.

Pour la réalisation de matériau hybride, une difficulté majeure est que les traitements thermiques à haute et moyenne température, c'est-à-dire supérieurs à 150°C sont à proscrire.

Or, dans les procédés décrits dans l'art antérieur et notamment dans Lin, S. et al., "Nanostructure evolution and calcium distribution in sol-gel derived bioactive glass". Journal of Materials Chemistry 2009, 19, (9), 1276-1282, ces traitements thermiques sont indispensables pour l'obtention d'un réseau vitreux homogène, notamment pour l'incorporation du calcium au sein du réseau silicate.

L'utilisation d'un précurseur alcoxyde pour le calcium permet l'incorporation du calcium dans la phase inorganique sans traitement thermique.

Cependant la très grande réactivité des alcoxydes de calcium vis-à-vis des réactions d'hydrolyse/condensation en présence d'eau font que le sol obtenu est très instable, la polymérisation sol-gel ayant lieu extrêmement rapidement, ce qui a rendu impossible jusqu'à ce jour sa manipulation en vue de réaliser un implant poreux et n'a pas permis non plus une bonne incorporation du calcium dans le réseau silicate. Ainsi, les inventeurs ont découvert qu'en limitant au maximum l'introduction d'eau dans le sol et en utilisant un précurseur alcoxyde différent de celui utilisé dans la littérature (Ramila A. et al., "Synthesis routes for bioactive sol-gel glasses : alkoxides versus nitrates". Chemistry of Materials 2002, 14, (12), 542-548) (à savoir le méthoxyethoxyde de calcium), il est possible d'augmenter grandement la stabilité du sol. Les réactions d'hydrolyse/condensation sont alors suffisamment lentes pour permettre une incorporation homogène du calcium dans le réseau silicate, tout en restant suffisamment rapides pour permettre la polymérisation de la phase inorganique. Dans l'exemple, les précurseurs alcoxydes de silicium et calcium sont mélangés ensemble dans une solution alcoolique légèrement acidifiée. De préférence, les précurseurs alcoxydes sont le tétraéthoxysilane et l'éthoxyde de calcium. Ensuite la gélatine préalablement dissoute est ajoutée à ce mélange pour l'obtention d'un sol hybride. Les seuls apports d'eau se font par l'intermédiaire de l'acide et de la solution de gélatine : ceci est suffisant pour permettre les réactions d'hydrolyse/condensation tout en les limitant fortement de manière à avoir un sol stable et manipulable entre quelques minutes et quelques heures suivant les proportions des réactifs.

Lors de l'élaboration du mélange hybride, il peut être avantageux d'ajouter un agent couplant, tel qu'un organo-alcoxysilane, au mélange.

En effet, deux classes d'implants hybrides organiques -inorganiques peuvent être réalisées, en fonction de la nature de l'interface qui associe les composantes organiques (polymère biocompatible) et inorganiques (verre bioactif). La classe I correspond à des systèmes hybrides dans lesquels les deux composantes interagissent par des liaisons faibles (liaisons hydrogène, Van der Waals, ou électrostatiques). Dans la classe II au contraire, les composantes organiques-inorganiques sont liées fortement par liaisons covalentes ou ionocovalentes. Ceci peut être obtenu au moyen d'un agent couplant.

Par exemple, l'agent couplant peut être simplement ajouté à la solution aqueuse du polymère biodégradable P, ici la gélatine. Le rôle de l'agent couplant est de fonctionnaliser la gélatine, en vue de permettre l'établissement de liaisons covalentes avec la phase inorganique (réseau silicate du verre bioactif). Dans le cas d'un mélange composite, le couplage permet d'obtenir des particules de verre bioactif liées en surface à la gélatine. Dans le cas d'un mélange hybride, un véritable copolymère organo-minéral (hybride de classe II) est obtenu. L'intérêt est de pouvoir maîtriser à façon la dégradabilité de l'implant composite ou hybride ainsi que sa tenue mécanique, en agissant simplement sur le nombre de liaisons chimiques créées entre phases organique et inorganique, ce nombre de liaisons chimiques créées étant lié à la quantité d'agent couplant introduite.

Un exemple d'agent couplant utilisé avec succès dans l'invention est le GPTMS (3-glycidoxypropyltriméthoxysilane), qui est soluble dans une solution aqueuse de gélatine.

On a obtenu un matériau d'implant constitué de 70% en masse de gélatine et de 30% en masse de verre bioactif avec une partie dense représentant 25% du volume de l'implant et une partie poreuse représentant 75% du volume de l'implant.

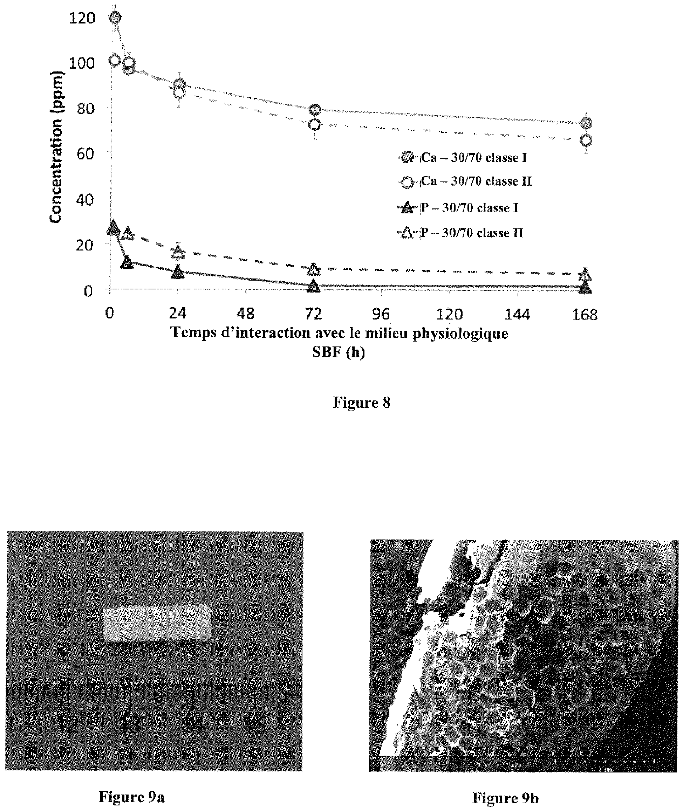

Ce matériau est montré en figures 9a et 9b.

Comme on peut le voir en figure 9a, le matériau obtenu a une taille de 2 cm x 0,5 cm très régulière et compacte.

Comme on le voit en figure 9b, cet implant est constitué d'une partie dense et d'une partie poreuse en superposition planaire.

### Exemple 2 :

Fabrication d'un matériau d'implant selon l'invention avec une matrice en matériau hybride avec une partie dense et une partie poreuse en superposition concentrique.

On a débuté par l'étape d'empilement compact des microsphères d'agent porogène en polyméthacrylate de méthyle dans un moule ayant la géométrie recherchée (diamètre de 6 mm) pour la partie poreuse de l'implant. Le matériau de l'agent porogène A était le polyméthacrylate de méthyle. Les sphères avaient un diamètre compris entre 200 et 400 µm. L'empilement a subi une fusion partielle des billes avec un mélange de solvant éthanol / acétone et avec une durée permettant la cohésion des billes. Après séchage de l'empilement compact de billes, l'empilement de billes est démoulé. Nous avons ainsi un bloc de billes cohésives. Ce bloc est placé au centre du moule ayant la géométrie recherchée pour l'implant (diamètre de 12 mm) et des dimensions supérieures au bloc de billes. Les dimensions de ce moule sont adaptées aux dimensions totales de l'ensemble de la partie dense et de la partie poreuse souhaité. Le bloc est fixé au fond et au centre du moule afin d'éviter son déplacement lors de l'infiltration.

Dans une deuxième étape, le mélange hybride de classe I a été versé dans le moule contenant l'empilement de billes. Le volume des billes d'agent porogène A est de 20%, par rapport au volume total du mélange agent porogène A-polymère biodégradable P-précurseurs alcoxydes du verre bioactif M. Une centrifugation ou une infiltration sous pression ou une infiltration sous vide peuvent être utilisées pour aider le mélange hybride à remplir les interstices entre les microsphères de polyméthacrylate de méthyle. Un bouchon, dont la surface est plane, est placé au contact du sol avec l'aide d'une légère pression dans le but d'obtenir une surface plane sur l'implant.

Le matériau hybride a été obtenu par un procédé sol-gel.

Dans ce procédé, un sol contenant tous les précurseurs alcoxydes du verre bioactif est amené à gélifier par une succession de réactions de polymérisation.

Les précurseurs alcoxydes étaient en des quantités telles que la composition du verre bioactif était 75% SiO₂ et 25% CaO, en masse, par rapport à la masse totale du verre bioactif obtenu au final. La composition finale de l'implant obtenu était 30% verre bioactif-70% gélatine en masse, par rapport à la masse totale du matériau d'implant.

Dans le cas du présent exemple, la gélatine (le polymère biodégradable P) a été ajoutée avant gélification du sol, de manière à obtenir un mélange hybride.

L'implant obtenu est représenté en figures 10 a et 10b.

Comme on peut le voir en figure 10a, l'implant a une forme cylindrique avec une partie dense sur la périphérie externe et une partie poreuse sur la périphérie interne.

L'implant est homogène et compact.

La figure 10b montre clairement la superposition concentrique de la partie dense et de la partie poreuse obtenues dans l'implant.

### Exemple 3 :

Fabrication d'un matériau d'implant selon l'invention avec une matrice en matériau hybride dont la porosité est dite « aléatoire ».

On a commencé par réaliser un mélange de billes de différents diamètres. Le mélange est constitué de 25% en masse de billes de 100-200µm de diamètre, de 25% de billes de 200-400µm de diamètre, de 25% de billes de 400-600µm de diamètre et de 25% de billes de 600-1000µm de diamètre. Ensuite, on a continué par l'étape d'empilement des microsphères d'agent porogène polyméthacrylate de méthyle dans un moule ayant la géométrie recherchée pour l'implant. Le volume des microsphères d'agent porogène A représentait 50% du volume total du mélange agent porogène A-polymère biodégradable P-précurseurs du verre bioactif M. Le matériau de l'agent porogène A était le poly(méthacrylate de méthyle).

Dans une deuxième étape, le mélange hybride de classe II a été versé dans le moule contenant l'empilement de billes. Le volume de mélange hybride est tel que la totalité du bloc de billes fusionnées est recouverte. Une centrifugation ou une infiltration sous pression ou une infiltration sous vide peuvent être utilisées pour aider le mélange hybride à remplir les interstices entre les microsphères de polyméthacrylate de méthyle. Un bouchon, dont la surface est plane, est placé au contact du sol avec l'aide d'une légère pression dans le but d'obtenir une surface plane sur l'implant.

Le matériau hybride a été obtenu par un procédé sol-gel.

Dans ce procédé, un sol contenant tous les précurseurs alcoxydes du verre bioactif est amené à gélifier par une succession de réactions de polymérisation.

Les précurseurs alcoxydes étaient en des quantités telles que la composition du verre bioactif était 75% SiO₂ et 25% CaO, en masse, par rapport à la masse totale du verre bioactif obtenu au final. La composition finale de l'implant obtenu était 30% verre bioactif - 70% gélatine en masse, par rapport à la masse totale du matériau d'implant.

Dans le cas du présent exemple, la gélatine (le polymère biodégradable P) a été ajoutée avant gélification du sol, de manière à obtenir un mélange hybride.

Comme on peut le voir en figures 11a et 11b, ce matériau est un implant dont la taille des pores varie de manière aléatoire au sein de l'implant.

Comme on le voit sur la figure 11a, le matériau d'implant de l'invention forme une seule pièce dont une partie est dense et l'autre poreuse.

La figure 11b est une vue prise au microscope électronique d'une coupe de l'implant obtenu à cet exemple sur laquelle on distingue nettement la partie poreuse dite « aléatoire » et la partie dense en superposition planaire.

### Exemple 4 :

Fabrication d'un matériau d'implant selon l'invention avec une matrice en matériau hybride avec une partie dense et une partie poreuse en superposition planaire dans lequel le polymère biodégradable P est du PDLLA.

On a procédé comme à l'exemple 1, sauf que l'on a remplacé la gélatine par du PDLLA.

La composition du verre bioactif était 75% SiO₂ - 25% CaO, en masse, par rapport à la masse totale du verre bioactif, et la composition finale de l'implant obtenu était 30% verre bioactif - 70% PDLLA, en masse, par rapport à la masse totale du matériau d'implant.

L'agent porogène A était des microsphères de paraffine d'un diamètre compris entre 600 et 1000 µm. Les sphères d'agent porogène A représentait 40% en volume, par rapport au volume total du mélange agent porogène A-polymère biodégradable P-précurseurs alcoxydes du verre bioactif M.

Le solvant S1 était du tétrahydrofurane.

Le solvant S était du cyclohexane.

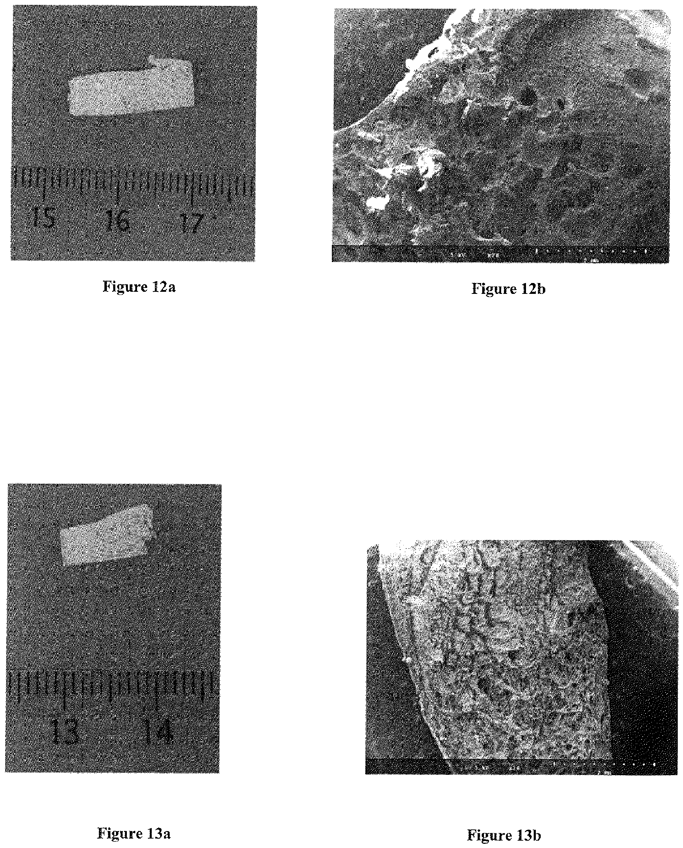

L'implant obtenu est montré en figure 12a sur laquelle on voit que le matériau d'implant a une taille d'environ 2 cm avec une partie poreuse représentant plus du tiers du matériau d'implant.

La figure 12b est une vue prise au microscope électronique d'une coupe de l'implant obtenu à cet exemple sur laquelle on distingue nettement la partie poreuse et la partie dense en superposition planaire.

### Exemple 5 :

Fabrication d'un matériau d'implant selon l'invention avec une matrice en matériau hybride avec une partie dense et une partie poreuse en superposition planaire dans lequel le polymère biodégradable P est du PCL.

On a procédé comme à l'exemple 1, sauf que l'on a remplacé la gélatine par du PCL.

La composition du verre bioactif était 75% SiO₂ - 25% CaO, en masse, par rapport à la masse totale du verre bioactif, et la composition finale de l'implant obtenu était 30% verre bioactif - 70% PCL, en masse, par rapport à la masse totale du matériau d'implant.

L'agent porogène A était des microsphères de paraffine d'un diamètre compris entre 600 et 1000µm. Les sphères d'agent porogène représentaient 40% en volume, par rapport au volume total du mélange agent porogène A-polymère biodégradable P-précurseurs alcoxydes du verre bioactif M.

Le solvant S1 était du tétrahydrofurane.

Le solvant S était du cyclohexane.

Comme on peut le voir en figures 13a et 13b, ce matériau d'implant a une partie poreuse et une partie en superposition planaire.

L'implant obtenu à cet exemple est montré aux figures 13a et 13b.

Comme on le voit en figure 13a, l'implant de l'invention a une partie dense de forme régulière et une partie poreuse moins compacte. Il a d'une manière générale une forme tronconique.

La figure 13b montre l'obtention d'une structure de l'implant dans laquelle une partie est dense et l'autre partie est poreuse en superposition planaire.

### Exemple 6 :

Fabrication d'un matériau d'implant selon l'invention avec une matrice en matériau hybride dont la porosité est dite « aléatoire » dans lequel le polymère biodégradable P est du PDLLA.

On a procédé comme à l'exemple 3, sauf que l'on a remplacé la gélatine par du PDLLA.

La composition du verre bioactif était 75% SiO₂ - 25% CaO, en masse, par rapport à la masse totale du verre bioactif, et la composition finale de l'implant obtenu était 30% verre bioactif - 70% PDLLA, en masse, par rapport à la masse totale du matériau d'implant.

L'agent porogène A était des microsphères de paraffine dont les diamètres sont compris entre 100-200 µm, 200-400 µm, 400-600 µm et 600-800 µm. Chaque distribution de taille représentait une fraction de 25% en masse de la masse totale des billes introduites dans le moule.

Les sphères d'agent porogène A représentaient 30% en volume, par rapport au volume total du mélange agent porogène A-polymère biodégradable P-précurseurs alcoxydes du verre bioactif M.

Le solvant S1 était du tétrahydrofurane.

Le solvant S était du cyclohexane.

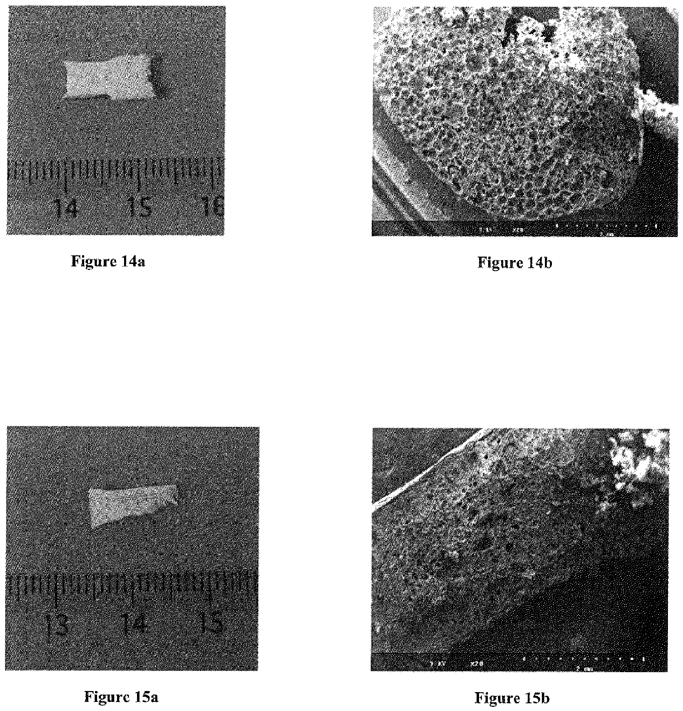

L'implant obtenu à cet exemple est montré aux figures 14a et 14b.

Comme on peut le voir en figures 14a et 14b, ce matériau est un implant dont la taille des pores varie de manière aléatoire au sein de l'implant, et comme on le voit sur la figure 14a, le matériau d'implant de l'invention forme une seule pièce dont une partie est dense et l'autre poreuse.

Comme on le voit sur la figure 14b, la porosité de la partie poreuse est aléatoire en ce sens que la distribution des pores est aléatoire.

### Exemple 7 :

Fabrication d'un matériau d'implant selon l'invention avec une matrice en matériau hybride dont la porosité est dite « aléatoire » dans lequel le polymère biodégradable P est du PCL.

On a procédé comme à l'exemple 3, sauf que l'on a remplacé la gélatine par du PCL.

La composition du verre bioactif était 75% SiO₂ - 25% CaO, en masse, par rapport à la masse totale du verre bioactif, et la composition finale de l'implant obtenu était 30% verre bioactif - 70% PCL, en masse, par rapport à la masse totale du matériau d'implant.

L'agent porogène A était des microsphères de paraffine dont les diamètres sont compris entre 100-200 µm, 200-400 µm, 400-600 µm et 600-800 µm. Chaque distribution de taille représentait une fraction de 25% en masse de la masse totale des billes introduites dans le moule.

Les sphères d'agent porogène A représentaient 50% en volume, par rapport au volume total du mélange agent porogène A-polymère biodégradable P-précurseurs alcoxydes du verre bioactif M.

Le solvant S1 était du tétrahydrofurane.

Le solvant S était du cyclohexane.

L'implant obtenu à cet exemple est montré aux figures 15a et 15b.

Comme on le voit sur la figure 15a, le matériau d'implant de l'invention forme une seule pièce dont une partie est dense et l'autre poreuse.

Comme on le voit sur la figure 15b, la porosité de la partie poreuse est aléatoire en ce sens que la distribution des pores est aléatoire.

Comme on peut le voir en figures 15a et 15b, ce matériau est un implant dont la taille des pores varie de manière aléatoire au sein de l'implant.

### Exemple 8 :

Fabrication d'un implant selon l'invention par utilisation d'une préforme obtenue par impression 3D.

La méthode de structuration par empreinte rigide est ici mise en œuvre, une préforme en ABS étant utilisée comme empreinte sacrificielle pour générer la porosité dans l'implant. Dans l'exemple, la préforme à géométrie cylindrique a été fabriquée par impression 3D et consistait en un maillage régulier de barreaux en ABS, comme visible en figure 5a. La préforme est d'abord introduite dans un moule, puis le sol hybride contenant le polymère biodégradable et les précurseurs alcoxydes du verre bioactif, le sol hybride emplissant alors les interstices de la préforme. Dans cet exemple, le polymère biodégradable était de la gélatine, et les précurseurs alcoxydes du verre bioactif étaient le tétraéthylorthosilicate et l'éthoxyde de calcium, mélangés dans des proportions telles que la composition du verre bioactif obtenue était 75%SiO₂ - 25% CaO. La composition finale de l'implant obtenu était 30% verre bioactif - 70% gélatine en masse, par rapport à la masse totale du matériau d'implant. De plus, un agent couplant, le GPTMS, avait été introduit dans le sol hybride de manière à lier les phases organiques et inorganiques et synthétiser ainsi un hybride de classe II.

Une centrifugation ou une infiltration sous pression ou une infiltration sous vide peuvent être utilisées pour aider le mélange hybride à remplir les interstices libres des murs d'ABS de la préforme.

Si l'on veut que l'implant possède une partie dense, il est nécessaire que la quantité de sol hybride introduite dans le moule soit supérieure au volume laissé vacant dans la préforme (espace interstitiel accessible entre les barreaux d'ABS de la préforme). Dans ce cas, un certain volume de sol hybride surnagera au-dessus de la préforme ; dans l'exemple, la hauteur de liquide surnageant était égale à la hauteur de la préforme. Le volume de la préforme en ABS représentait 30% du volume total du mélange agent porogène A-polymère biodégradable P-précurseurs alcoxydes du verre bioactif M.

Après gélification complète et séchage à l'ambiante, le mélange est lavé plusieurs fois dans des bains d'acétone, qui est un solvant de l'ABS, afin de dissoudre intégralement la préforme sans dégrader le matériau hybride.

Comme on le voit en figure 5b et en figure 5c, l'implant obtenu est la réplique inverse exacte de la préforme de départ et est constitué d'un maillage de murs hybrides en bioverre-gélatine régulièrement espacés. Dans l'exemple, les murs obtenus avaient une épaisseur moyenne de 150 microns et leur espacement moyen était de 450 microns, mais ces caractéristiques peuvent évidemment être variées à façon puisque dépendant directement du maillage initial de la préforme.

La figure 5b montre également qu'une partie dense (2000) est obtenue avec succès à l'une des extrémités de l'implant.

### Exemple 9 :

Fabrication d'un matériau d'implant selon l'invention avec une matrice en matériau hybride dans laquelle la taille des pores de la partie poreuse croit de la base de l'implant vers le haut de l'implant et se termine par une partie dense dans lequel le polymère biodégradable P est du PCL.

On a réalisé un empilement compact de sphères d'agent porogène en trois opérations successives, de sorte à avoir une répartition stratifiée par intervalle de tailles de sphères. Le volume des sphères d'agent porogène a été fractionné en trois tiers, en fonction de la taille des sphères. On a débuté par l'étape d'empilement compact des microsphères d'agent porogène en paraffine de diamètre 100-200 µm, la quantité introduite représentant un tiers du volume des sphères. Ensuite, on a introduit par dessus des sphères de diamètre 400-600 µm, représentant un autre tiers du volume total des sphères. Enfin, le dernier tiers du volume total des sphères est ajouté par dessus, et correspond à des sphères dont le diamètre est supérieur à 600 µm. Le matériau de l'agent porogène A était la paraffine. La totalité des sphères porogènes introduites représentait 45% en volume, par rapport au volume total du mélange agent porogène A - polymère biodégradable P - précurseurs alcoxydes du verre bioactif M.

Dans une deuxième étape, le mélange hybride de classe I bioverre/PCL d'un rapport 30/70 en masse a été versé dans le moule contenant l'empilement de billes. La composition du verre bioactif était 75% SiO₂ - 25% CaO, en masse, par rapport à la masse totale du verre bioactif, et la composition finale du sol hybride était de 30% verre bioactif - 70% PCL, en masse, par rapport à la masse totale du sol hybride.

Le solvant S1 était du tétrahydrofurane.

Le solvant S était du cyclohexane.

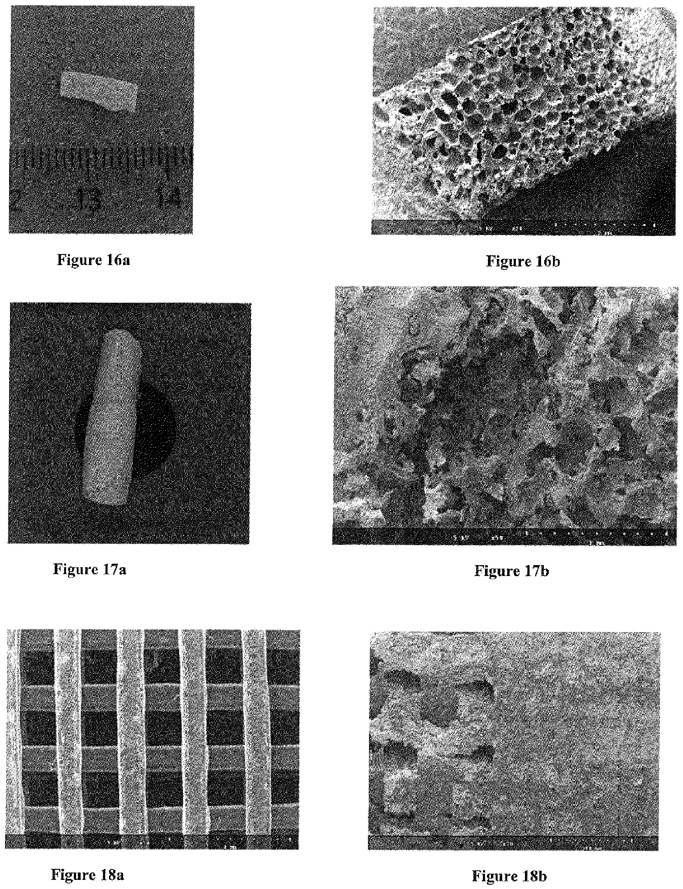

L'implant obtenu à cet exemple est montré aux figures 16a et 16b.

Comme on le voit sur la figure 16a, le matériau d'implant de l'invention forme une seule pièce dont une partie est dense et l'autre poreuse.

Comme on le voit sur la figure 16b, la porosité de la partie poreuse est graduelle en ce sens que la distribution des pores croît de manière stratifiée jusqu'à la partie dense de l'implant (porosité graduelle).

Comme on peut le voir en figures 16a et 16b, ce matériau est un implant dont la taille des pores varie de manière graduelle au sein de l'implant.

Ainsi, les différents matériaux d'implants comprenant une partie dense et une partie poreuse sont obtenus grâce à l'invention.

Il apparaîtra clairement à l'homme de l'art que ces matériaux, bien que fabriqués dans un moule à la taille et à la forme du défaut osseux à combler ou à régénérer, pourront être usinés pour être ajustés encore plus précisément et que cette étape d'usinage fait également partie du procédé de l'invention.

### Exemple 10 :

Fabrication d'un matériau d'implant selon l'invention avec une matrice en matériau hybride avec une composition de bioverre de 75% SiO₂, 20% CaO et 5% SrO et dont la porosité est dite « aléatoire » dans lequel le polymère biodégradable P est du PCL.

On a procédé comme à l'exemple 7, sauf que l'on a modifié les quantités de précurseurs de telle sorte à obtenir une composition de bioverre égale à 75% SiO₂, 20% CaO et 5%SrO, en masse, par rapport à la masse totale du verre bioactif et on a modifié le rapport verre bioactif sur polymère pour obtenir un rapport 40% verre bioactif - 60% PCL, en masse, par rapport à la masse totale du matériau d'implant. Pour réaliser cette composition de bioverre, on a utilisé en plus du calcium éthoxyde et du tétraéthylorthosilicate, un précurseur alcoxyde de strontium le strontium isopropoxyde. La composition du verre bioactif était 75% SiO₂, 20% CaO et 5% SrO, en masse, par rapport à la masse totale du verre bioactif, et la composition finale de l'implant obtenu était 40% verre bioactif - 60% PCL, en masse, par rapport à la masse totale du matériau d'implant.

L'agent porogène A était des microsphères de paraffine dont les diamètres sont compris entre 200-400 µm, 400-600 µm et 600-800 µm. Chaque distribution de taille représentait une fraction de 33% en masse de la masse totale des billes introduites dans le moule.

Les sphères d'agent porogène A représentaient 30% en volume, par rapport au volume total du mélange agent porogène A-polymère biodégradable P-précurseurs alcoxydes du verre bioactif M.

Le solvant S1 était du tétrahydrofurane.

Le solvant S était du cyclohexane.

L'implant obtenu à cet exemple est montré aux figures 17a et 17b.

Comme on le voit sur les figures 17a et 17b, le matériau d'implant de l'invention forme une seule pièce dont une partie est dense et l'autre poreuse.

Comme on peut le voir en figure 17b, ce matériau est un implant dont la taille des pores varie de manière aléatoire au sein de l'implant.

### Exemple 11 :

Fabrication d'un implant selon l'invention par utilisation d'une préforme obtenue par impression 3D.

On a procédé comme à l'exemple 8, sauf que l'on a remplacé la gélatine par du collagène, l'agent couplant GPTMS a été remplacé par du GPTES et la préforme en ABS a été remplacée par une préforme en PLA.

La composition du verre bioactif était 75% SiO₂ , 25% CaO , en masse, par rapport à la masse totale du verre bioactif, et la composition finale de l'implant obtenu était 30% verre bioactif - 70% collagène, en masse, par rapport à la masse totale du matériau d'implant.

L'agent porogène A était une préforme en PLA dont les pores ont une taille d'environ 200 µm et les barreaux ont une taille d'environ 300 µm.

La préforme A représentait 40% en volume, par rapport au volume total du mélange agent porogène A-polymère biodégradable P-précurseurs alcoxydes du verre bioactif M.

La figure 18a montre une coupe de la préforme utilisée lors de la fabrication du matériau d'implant.

Le solvant S1 était l'acide chlorhydrique 10mM.

Le solvant S était du chloroforme.

L'implant obtenu à cet exemple est montré à la figure 18b.

Comme on le voit sur la figure 18b, le matériau d'implant de l'invention forme une seule pièce dont une partie est dense et l'autre poreuse. La partie poreuse de l'implant est la réplique inverse de la préforme utilisée.

### Exemple 12 :

Fabrication d'un implant selon l'invention par utilisation d'une préforme obtenue par impression 3D.

On a procédé comme à l'exemple 8, sauf que l'on a remplacé la préforme en ABS par une préforme en PLA et l'agent couplant GPTMS a été remplacé par du GPTES.

La composition du verre bioactif était 75% SiO₂, 25% CaO, en masse, par rapport à la masse totale du verre bioactif, et la composition finale de l'implant obtenu était 30% verre bioactif - 70% gélatine, en masse, par rapport à la masse totale du matériau d'implant.

L'agent porogène A était une préforme en PLA dont les pores ont une taille d'environ 300 µm et les barreaux ont une taille d'environ 200 µm.

La préforme en PLA représentait 50% en volume, par rapport au volume total du mélange agent porogène A-polymère biodégradable P-précurseurs alcoxydes du verre bioactif M.

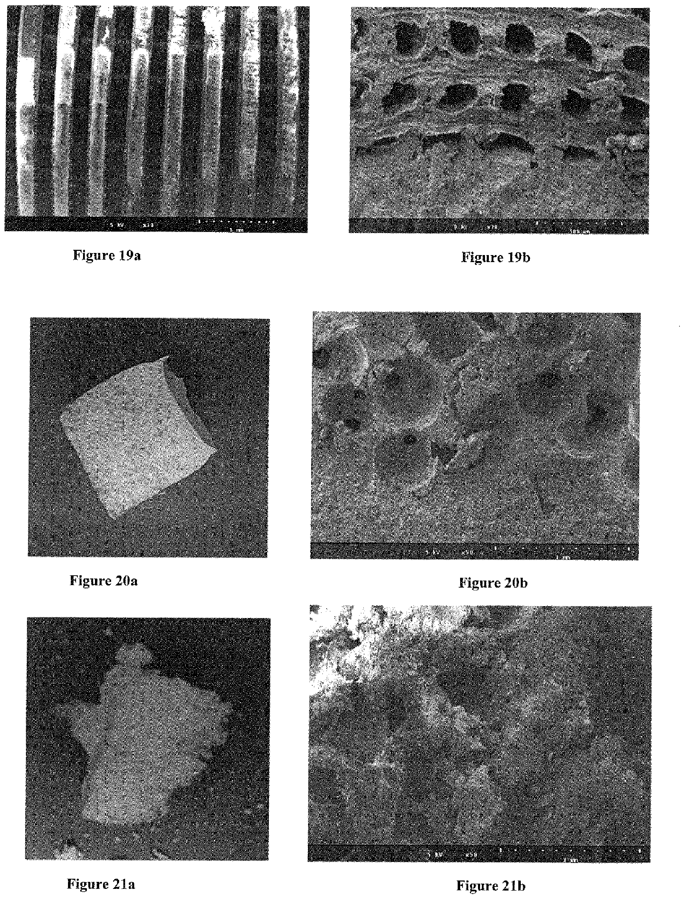

La figure 19a montre une coupe de la préforme utilisée lors de la fabrication du matériau d'implant.

Le solvant S1 était de l'eau désionisée.

Le solvant S était du chloroforme.

L'implant obtenu à cet exemple est montré à la figure 19b.

Comme on le voit sur la figure 19b, le matériau d'implant de l'invention forme une seule pièce dont une partie est dense et l'autre poreuse. La partie poreuse de l'implant est la réplique inverse de la préforme utilisée.

### Exemple 13 :

Fabrication d'un matériau d'implant selon l'invention avec une matrice en matériau hybride avec une composition de bioverre de 75% SiO₂, 20% CaO et 5% SrO et dont la porosité est dite « aléatoire » dans lequel le polymère biodégradable P est de la gélatine.

On a procédé comme à l'exemple 3, sauf que l'on a modifié les quantités de précurseurs de telle sorte à obtenir une composition de bioverre égale à 75% SiO₂, 20% CaO et 5% SrO, en masse, par rapport à la masse totale du verre bioactif et l'agent couplant GPTMS a été remplacé par du GPTES. Pour réaliser cette composition de bioverre, on a utilisé en plus du calcium éthoxyde et du tétraéthylorthosilicate, un précurseur alcoxyde de strontium le strontium isopropoxyde.La composition du verre bioactif était 75% SiO₂, 20% CaO et 5% SrO, en masse, par rapport à la masse totale du verre bioactif, et la composition finale de l'implant obtenu était 30% verre bioactif - 70% gélatine, en masse, par rapport à la masse totale du matériau d'implant.

L'agent porogène A était des microsphères de polyméthacrylate de méthyle dont les diamètres sont compris entre 200-400 µm, 400-600 µm et 600-800 µm. Chaque distribution de taille représentait une fraction de 33% en masse de la masse totale des billes introduites dans le moule.

Les sphères d'agent porogène A représentaient 50% en volume, par rapport au volume total du mélange agent porogène A-polymère biodégradable P-précurseurs alcoxydes du verre bioactif M.

Le solvant S1 était de l'eau désionisée.

Le solvant S était de l'acétone.

L'implant obtenu à cet exemple est montré aux figures 20a et 20b.

La figure 20a montre une photographie du matériau d'implant.

La figure 20b montre une photographie prise au microscope électronique à balayage à un grandissement de x50 d'une coupe de l'implant en un matériau hybride, à l'interface dense/poreux.

Comme on le voit sur les figures 20a et 20b, le matériau d'implant de l'invention forme une seule pièce dont une partie est dense et l'autre poreuse.

Comme on peut le voir en figure 20b, ce matériau est un implant dont la taille des pores varie de manière aléatoire au sein de l'implant.

### Exemple 14 :

Fabrication d'un matériau d'implant selon l'invention avec une matrice en matériau hybride avec une partie dense et une partie poreuse en superposition planaire dans lequel le polymère biodégradable P est de l'acide hyaluronique.

On a procédé comme à l'exemple 1, sauf que l'on a remplacé la gélatine par de l'acide hyaluronique l'agent couplant GPTMS a été remplacé par du GPTES (du 3-glycidoxypropyltriéthoxysilane).

La composition du verre bioactif était 75% SiO₂ et 25% CaO, en masse, par rapport à la masse totale du verre bioactif, et la composition finale de l'implant obtenu était 30% verre bioactif - 70% acide hyaluronique, en masse, par rapport à la masse totale du matériau d'implant.

L'agent porogène A était des microsphères de polyméthacrylate de méthyle dont les diamètres sont compris entre 400-600 µm.

Les sphères d'agent porogène A représentaient 40% en volume, par rapport au volume total du mélange agent porogène A-polymère biodégradable P-précurseurs alcoxydes du verre bioactif M.

Le solvant S1 était de l'eau désionisée.

Le solvant S était de l'acétone.

L'implant obtenu à cet exemple est montré aux figures 21a et 21b.

Comme on le voit sur les figures 21a et 21b, le matériau d'implant de l'invention forme une seule pièce dont une partie est dense et l'autre poreuse.

Comme on peut le voir en figure 21b, ce matériau est un implant dont la taille des pores varie de manière aléatoire au sein de l'implant.

## Revendications

1. Matériau d'implant en un matériau hybride, ledit matériau hybride comprenant :
- un polymère biodégradable P soluble dans au moins un solvant S1 et insoluble dans au moins un solvant S différent du solvant S1 et un verre bioactif à base de SiO₂ et CaO, contenant optionnellement P₂O₅ et/ou optionnellement dopé au strontium,
**caractérisé en ce qu'**il comprend la superposition de :
- une partie poreuse ayant plus de 90% en nombre, de pores dont la plus grande dimension est supérieure ou égale à 100 µm, et
- une partie dense (2, 20, 200, 2000, 20000) ayant plus de 80% en nombre, de pores dont la plus grande dimension est inférieure à 50 µm.

2. Matériau d'implant selon la revendication 1, **caractérisé en ce que** le rapport volume partie dense/volume partie poreuse est compris entre 10/90 et 90/10.

3. Matériau d'implant selon la revendication 1 ou 2, **caractérisé en ce que** les pores de la partie poreuse (1, 100, 1000, 10000) ont tous la même forme et les mêmes dimensions.

4. Matériau d'implant selon la revendication 1 ou 2, **caractérisé en ce que** la partie poreuse (10) a des pores (30) dont la plus grande dimension décroit de la base de l'implant vers la partie dense (20).

5. Matériau d'implant selon la revendication 1 ou 2, **caractérisé en ce que** la partie poreuse a des pores dont la plus grande dimension croit de la base de l'implant vers la partie dense.

6. Matériau d'implant selon l'une quelconque des revendications précédentes, **caractérisé en ce que** la partie poreuse (1, 10, 100, 1000) et la partie dense (2, 20, 200, 2000) sont en superposition planaire.

7. Matériau d'implant selon l'une quelconque des revendications 1 à 5, **caractérisé en ce que** la partie poreuse (10000) et la partie dense (20000) sont en superposition concentrique.

8. Matériau d'implant selon l'une quelconque des revendications précédentes, **caractérisé en ce que** les pores de la partie poreuse ont une forme sphérique.

9. Matériau d'implant selon l'une quelconque des revendications 1 à 7, **caractérisé en ce que** les pores de la partie poreuse ont la forme de polygones, de préférence la forme de carrés.

10. Procédé de fabrication d'un matériau d'implant selon l'une quelconque des revendications 1 à 8, **caractérisé en ce qu'**il comprend les étapes suivantes :
a) sélection des précurseurs alcoxydes d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S différent du solvant S1,
c) sélection de microsphères d'un agent porogène A ayant des diamètres et des tailles correspondant aux diamètres et tailles recherchés des pores dans le matériau constituant l'implant à fabriquer, cet agent porogène A étant :
- en un polymère insoluble dans le au moins un solvant S1 et soluble dans le au moins un solvant S,
le au moins un solvant S dans lequel le matériau du polymère biodégradable P est insoluble et le au moins un solvant S dans lequel le matériau de l'agent porogène A est soluble étant identiques,
d) introduction des microsphères de l'agent porogène A dans un moule ayant la forme et la taille recherchées pour l'implant, ces microsphères formant un empilement compact correspondant à la taille et à la forme des pores à obtenir pour la partie poreuse (1, 10, 100, 1000, 10000) du matériau d'implant, et représentant entre 5% et 50% en volume, par rapport au volume total du mélange agent porogène A - polymère biodégradable P - précurseurs alcoxydes du verre bioactif M,
e) introduction du polymère biodégradable P dans les précurseurs alcoxydes du verre bioactif M,
f) introduction du mélange obtenu à l'étape e) dans le moule, en une quantité supérieure au volume laissé vacant par les billes d'agent porogène A, afin d'obtenir un matériau d'implant comprenant la superposition d'une région poreuse et d'une région dense,
g) gélification du mélange contenu dans le moule après l'étape f),
h) démoulage du mélange obtenu à l'étape g),
i) élimination des microsphères d'agent porogène A par lavage avec le solvant S.

11. Procédé de fabrication d'un matériau d'implant selon l'une quelconque des revendications 1 à 9, **caractérisé en ce qu'**il comprend les étapes suivantes :
a) sélection des précurseurs alcoxydes d'un verre bioactif M à base de SiO₂ et CaO, contenant optionnellement P₂O₅ et/ou optionnellement dopé au strontium,
b) sélection d'un polymère biodégradable P qui est soluble dans au moins un solvant S1 et insoluble dans au moins un solvant S différent du solvant S1,
c) fabrication, par impression 3D, d'une préforme (9), en un polymère insoluble dans le au moins un solvant S1 est soluble dans le au moins un solvant S, cette préforme ayant la forme et la taille finales voulues pour les pores (3, 30) dans la partie poreuse (1, 10, 100, 1000, 10000) de l'implant final et représentant entre 5% et 50% en volume du volume total du mélange préforme (9) - polymère biodégradable P - précurseur alcoxydes du verre bioactif M,
d) introduction de la préforme (9) dans un moule ayant la forme et la taille recherchées de l'implant final,
e) introduction du polymère biodégradable P dans les précurseurs alcoxydes du verre bioactif M,
f) introduction du mélange obtenu à l'étape e) dans le moule, en une quantité supérieure au volume laissé vacant par la préforme (9), afin d'obtenir un matériau d'implant comprenant la superposition d'une région poreuse et d'une région dense,
g) gélification du mélange contenu dans le moule après l'étape f),
h) démoulage du mélange obtenu à l'étape g),
i) élimination de la préforme (9) par lavage avec le solvant S.

12. Procédé selon la revendication 10 ou 11, **caractérisé en ce que** les étapes e) et/ou f) sont mises en œuvre avant l'étape d).

13. Procédé selon la revendication 11 ou 12, caractérisé en ce les étapes d), e) et f) sont mises en œuvre simultanément.

14. Procédé selon l'une quelconque des revendications 10 à 13, **caractérisé en ce qu'**à l'étape d), l'empilement compact de microsphères ou la préforme (9) sont placés de façon à toucher les parois latérales du moule, en laissant un espace libre au dessus de l'empilement de microsphères ou de la préforme (9), ce par quoi la partie poreuse (1, 10, 100, 1000) et la partie dense du matériau d'implant (2, 20, 200, 2000) sont en superposition planaire.

15. Procédé selon l'une quelconque des revendications 10 à 13, **caractérisé en ce qu'**à l'étape d), l'empilement compact de microsphères ou la préforme (9) sont placés au centre du moule en laissant un espace libre entre l'empilement compact de microsphères ou les parois latérales de la préforme (9) et les parois latérales du moule, ce par quoi la partie poreuse (10000) et la partie dense du matériau d'implant (20000) sont en superposition concentrique.

16. Procédé selon l'une quelconque des revendications 10 à 15, **caractérisé en ce que** le polymère biodégradable P est un polymère biodégradable soluble dans au moins un solvant S1 et insoluble dans au moins un solvant S choisi parmi :
- les polysaccharides biorésorbables, de préférence choisis parmi le dextran, l'acide hyaluronique, l'agar, le chitosane, l'acide alginique, l'alginate de sodium ou de potassium, le galactomannane, le carraghénane, la pectine,
- les polyesters biorésorbables, de préférence l'alcool polyvinylique ou le poly(acide lactique),
- les polymères synthétiques biodégradables, de préférence un polyéthylène glycol, ou le poly(caprolactone),
- les protéines, de préférence la gélatine ou le collagène,
et **en ce que** le matériau de l'agent porogène ou de la préforme (9) est un matériau choisi parmi les polymères biodégradables insolubles dans le au moins un solvant S1 et solubles dans le au moins un solvant S, de préférence choisi parmi les polyméthacrylates d'alkyle en C₁ à C₄, de préférence le polyméthacrylate de méthyle ou le polyméthacrylate de butyle, le polyuréthane, l'acide polyglycolique, les différentes formes d'acides polylactiques, les copolymères d'acides lactique-coglycoliques, le polycaprolactone, le polypropylène fumarate, la paraffine et le naphtalène, ou l'acrylonitrile butadiène styrène (ABS),
le matériau de l'agent porogène A ou de la préforme (9) étant différent du polymère biodégradable P.

17. Procédé selon l'une quelconque des revendications 10 à 16, **caractérisé en ce que** le rapport en poids polymère biodégradable P/verre bioactif M est compris entre 20/80 et 80/20, bornes incluses.

18. Procédé selon l'une quelconque des revendications 11 à 17, **caractérisé en ce que** le verre bioactif M est un verre à base de SiO₂ et de CaO, le polymère biodégradable P est la gélatine, le matériau de la préforme (9) est l'ABS et le solvant S est l'acétone.

19. Procédé selon l'une quelconque des revendications 10 et 12 à 17, **caractérisé en ce que** le verre bioactif M est un verre à base de SiO₂ et de CaO, le polymère biodégradable P est la gélatine, le matériau de l'agent porogène A est le polyméthacrylate de méthyle et le solvant S est l'acétone.

20. Procédé selon l'une quelconque des revendications 10 à 19, **caractérisé en ce qu'**il comprend de plus, à l'étape f), une étape d'introduction d'un agent de couplage, de préférence un composé organoalkoxysilane, plus préférablement du 3-glycidoxypropyltriméthoxysilane (GPTMS), encore plus préférablement du 3-glycidoxypropyltriéthoxysilane (GPTES).

21. Implant en un matériau hybride pour le comblement de défauts osseux, la régénération osseuse et l'ingénierie tissulaire de l'os, **caractérisé en ce qu'**il comprend un matériau d'implant selon l'une quelconque des revendications 1 à 9 ou obtenu par le procédé selon l'une quelconque des revendications 10 à 20.

## Patentansprüche

1. Implantatmaterial aus einem Hybridmaterial, wobei das Hybridmaterial Folgendes umfasst:
- ein biologisch abbaubares Polymer P, das in mindestens einem Lösemittel S1 löslich ist, und in mindestens einem Lösemittel S, das von dem Lösemittel S1 unterschiedlich ist, nicht löslich ist, und ein bioaktives Glas auf der Basis von SiO₂ und CaO, das optional P₂O₅ enthält und/oder optional mit Strontium dotiert ist,
**dadurch gekennzeichnet, dass** es die Überlagerung umfasst aus:
- einem porösen Teil, der mehr als 90 Anzahl-% an Poren aufweist, deren größtes Maß größer oder gleich 100 µm ist, und
- einem dichten Teil (2, 20, 200, 2000, 20000), der mehr als 80 Anzahl-% an Poren aufweist, deren größtes Maß kleiner als 50 µm ist.

2. Implantatmaterial nach Anspruch 1, **dadurch gekennzeichnet, dass** das Verhältnis Volumen des dichten Teils/Volumen des porösen Teils zwischen 10:90 und 90:10 liegt.

3. Implantatmaterial nach Anspruch 1 oder 2, **dadurch gekennzeichnet, dass** die Poren des porösen Teils (1, 100, 1000, 10000) alle die gleiche Form und die gleichen Maße aufweisen.

4. Implantatmaterial nach Anspruch 1 oder 2, **dadurch gekennzeichnet, dass** der poröse Teil (10) Poren (30) aufweist, deren größtes Maß von der Basis des Implantats zu dem dichten Teil (20) abnimmt.

5. Implantatmaterial nach Anspruch 1 oder 2, **dadurch gekennzeichnet, dass** der poröse Teil Poren aufweist, deren größtes Maß von der Basis des Implantats zu dem dichten Teil zunimmt.

6. Implantatmaterial nach einem der vorstehenden Ansprüche, **dadurch gekennzeichnet, dass** der poröse Teil (1, 10, 100, 1000) und der dichte Teil (2, 20, 200, 2000) in planarer Überlagerung sind.

7. Implantatmaterial nach einem der Ansprüche 1 bis 5, **dadurch gekennzeichnet, dass** der poröse Teil (10000) und der dichte Teil (20000) in konzentrischer Überlagerung sind.

8. Implantatmaterial nach einem der vorstehenden Ansprüche, **dadurch gekennzeichnet, dass** die Poren des porösen Teils eine sphärische Form aufweisen.

9. Implantatmaterial nach einem der Ansprüche 1 bis 7, **dadurch gekennzeichnet, dass** die Poren des porösen Teils die Form von Vielecken, bevorzugt die Form von Quadraten aufweisen.

10. Herstellungsverfahren eines Implantatmaterials nach einem der Ansprüche 1 bis 8, **dadurch gekennzeichnet, dass** es die folgenden Schritte umfasst:
a) Auswahl der Alkoxidvorläufer eines bioaktiven Glases M auf der Basis von SiO₂ und CaO, das optional P₂O₅ enthält und/oder optional mit Strontium dotiert ist,
b) Auswahl eines biologisch abbaubaren Polymers P, das in mindestens einem Lösemittel S1 löslich ist und in mindestens einem Lösemittel S, das von dem Lösemittel S1 unterschiedlich ist, nicht löslich ist,
c) Auswahl von Mikrokügelchen eines Porenbildners A, die Durchmesser und Größen aufweisen, die den angestrebten Durchmessern und Größen der Poren in dem Material, das das herzustellende Implantat bildet, entsprechen, wobei dieser Porenbildner A:
- aus einem Polymer besteht, das in dem mindestens einen Lösemittel S1 nicht löslich ist, und in dem mindestens einen Lösemittel S löslich ist,
wobei das mindestens eine Lösemittel S, in dem das Material des biologisch abbaubaren Polymers P nicht löslich ist, und das mindestens eine Lösemittel S, in dem das Material des Porenbildners A löslich ist, identisch sind,
d) Einführen der Mikrokügelchen des Porenbildners A in eine Form, die die Form und die Größe aufweist, die für das Implantat angestrebt werden, wobei diese Mikrokügelchen eine kompakte Stapelung bilden, die der Größe und der Form der zu erzielenden Poren für den porösen Teil (1, 10, 100, 1000, 10000) des Implantatmaterials entspricht, und zwischen 5 Vol.-% und 50 Vol.-% in Bezug auf das Gesamtvolumen des Gemischs aus Porenbildner A - biologisch abbaubarem Polymer P - Alkoxidvorläufern des bioaktiven Glases M darstellt,
e) Einführen des biologisch abbaubaren Polymers P in die Alkoxidvorläufer des bioaktiven Glases M,
f) Einführen des Gemischs, das bei dem Schritt e) erhalten wird, in die Form mit einer Menge größer als das Volumen, das von den Kugeln des Porenbildners A belassen wird, um ein Implantatmaterial zu erhalten, das die Überlagerung eines porösen Bereichs und eines dichten Bereichs umfasst,
g) Gelieren des Gemischs, das in der Form nach dem Schritt f) enthalten ist,
h) Auslösen des Gemischs, das bei Schritt g) erhalten wird,
i) Eliminieren der Mikrokügelchen aus Porenbildner A durch Waschen mit dem Lösemittel S.

11. Herstellungsverfahren eines Implantatmaterials nach einem der Ansprüche 1 bis 9, **dadurch gekennzeichnet, dass** es die folgenden Schritte umfasst:
a) Auswahl der Alkoxidvorläufer eines bioaktiven Glases M auf der Basis von SiO₂ und CaO, das optional P₂O₅ enthält und/oder optional mit Strontium dotiert ist,
b) Auswahl eines biologisch abbaubaren Polymers P, das in mindestens einem Lösemittel S1 löslich ist und in mindestens einem Lösemittel S, das von dem Lösemittel S1 unterschiedlich ist, nicht löslich ist,
c) Herstellen durch 3D-Druck einer Vorform (9) aus einem Polymer, das in dem mindestens einen Lösemittel S1 nicht löslich ist und in dem mindestens einen Lösemittel S löslich ist, wobei diese Vorform die abschließende Form und Größe, die für die Poren (3, 30) in dem porösen Teil (1, 10, 100, 1000, 10000) des abschließenden Implantats gewünscht werden, aufweist und zwischen 5 Vol.-% und 50 Vol.-% des Gesamtvolumens des Gemischs der Vorform (9) - biologisch abbaubares Polymer P - Alkoxidvorläufer des bioaktiven Glases M darstellt,
d) Einführen der Vorform (9) in eine Form, die die Form und die Größe aufweist, die für das abschließende Implantat angestrebt werden,
e) Einführen des biologisch abbaubaren Polymers P in die Alkoxidvorläufer des bioaktiven Glases M,
f) Einführen des Gemischs, das bei dem Schritt e) erhalten wird, in die Form in einer Menge, die größer ist als das Volumen, das von der Vorform (9) leer gelassen wird, um ein Implantatmaterial zu erhalten, das die Überlagerung eines porösen Bereichs und eines dichten Bereichs umfasst,
g) Gelieren des Gemischs, das in der Form nach dem Schritt f) enthalten ist,
h) Auslösen des Gemischs, das bei dem Schritt g) erhalten wird,
i) Eliminieren der Vorform (9) durch Waschen mit dem Lösemittel S.

12. Verfahren nach Anspruch 10 oder 11, **dadurch gekennzeichnet, dass** die Schritte e) und/oder f) vor dem Schritt d) umgesetzt werden.

13. Verfahren nach Anspruch 11 oder 12, **dadurch gekennzeichnet, dass** die Schritte d), e) und f) gleichzeitig umgesetzt werden.

14. Verfahren nach einem der Ansprüche 10 bis 13, **dadurch gekennzeichnet, dass** bei dem Schritt d) die kompakte Stapelung aus Mikrokügelchen oder die Vorform (9) derart platziert wird, dass sie die Seitenwände der Form berührt, indem ein freier Raum oberhalb der Stapelung aus Mikrokügelchen oder der Vorform (9) belassen wird, wodurch der poröse Teil (1, 10, 100, 1000) und der dichte Teil des Implantatmaterials (2, 20, 200, 2000) in planarer Überlagerung sind.

15. Verfahren nach einem der Ansprüche 10 bis 13, **dadurch gekennzeichnet, dass** bei dem Schritt d) die kompakte Stapelung aus Mikrokügelchen oder die Vorform (9) in der Mitte der Form platziert wird, indem ein freier Raum zwischen der kompakten Stapelung aus Mikrokügelchen oder den Seitenwänden der Vorform (9) und den Seitenwänden der Form belassen wird, wodurch der poröse Teil (10000) und der dichte Teil des Implantatmaterials (20000) in konzentrischer Überlagerung sind.

16. Verfahren nach einem der Ansprüche 10 bis 15, **dadurch gekennzeichnet, dass** das biologisch abbaubare Polymer P ein biologisch abbaubares Polymer ist, das in mindestens einem Lösemittel S1 löslich ist und in mindestens einem Lösemittel S nicht löslich ist, das ausgewählt ist aus:
- den bioresorbierbaren Polysacchariden, bevorzugt ausgewählt aus Dextran, Hyaluronsäure, Agar, Chitosan, Alginsäure, Natrium- oder Kaliumalginat, Galactomannan, Carrageen, Pektin,
- den bioresorbierbaren Polyestern, bevorzugt Polyvinylalkohol oder Poly(milchsäure),
- den synthetischen biologisch abbaubaren Polymeren, bevorzugt einem Polyethylenglycol oder Poly(caprolacton),
- den Proteinen, bevorzugt Gelatine oder Kollagen,
und dass das Material des Porenbildners oder der Vorform (9) ein Material ist, das aus den biologisch abbaubaren Polymeren, die in dem mindestens einen Lösemittel S1 nicht löslich sind und in dem mindestens einen Lösemittel S löslich sind, ausgewählt ist, bevorzugt aus den Alkylpolymethacrylaten in C₁ bis C₄, bevorzugt Methylpolymethacrylat oder Butylpolymethacrylat, Polyurethan, Polyglycolsäure, den unterschiedlichen Formen von Polymilchsäuren, den Copolymeren von Milchsäure-Co-Glycolsäuren, Polycaprolacton, Polypropylenfumarat, Paraffin und Naphthalin oder Acrylnitril-Butadien-Styrol (ABS),
wobei das Material des Porenbildners A oder der Vorform (9) von dem biologisch abbaubaren Polymer P unterschiedlich ist.

17. Verfahren nach einem der Ansprüche 10 bis 16, **dadurch gekennzeichnet, dass** das Gewichtsverhältnis biologisch abbaubares Polymer P/bioaktives Glas M zwischen einschließlich 20:80 und 80:20 umfasst ist.

18. Verfahren nach einem der Ansprüche 11 bis 17, **dadurch gekennzeichnet, dass** das bioaktive Glas M ein Glas auf der Basis von SiO₂ und CaO ist, das biologisch abbaubare Polymer P Gelatine ist, das Material der Vorform (9) ABS ist, und das Lösemittel S Aceton ist.

19. Verfahren nach einem der Ansprüche 10 und 12 bis 17, **dadurch gekennzeichnet, dass** das bioaktive Glas M ein Glas auf der Basis von SiO₂ und von CaO ist, das biologisch abbaubare Polymer P Gelatine ist, das Material des Porenbildners A Methylpolymethacrylat ist und das Lösemittel S Aceton ist.

20. Verfahren nach einem der Ansprüche 10 bis 19, **dadurch gekennzeichnet, dass** es außerdem bei dem Schritt f) einen Schritt des Einführens eines Kopplungsmittels umfasst, bevorzugt eines Organoalkoxysilans, bevorzugter des 3-Glycidoxypropyltrimethoxysilans (GPTMS), noch bevorzugter des 3-Glycidoxypropyltriethoxysilans (GPTES).

21. Implantat aus einem Hybridmaterial für das Auffüllen von Knochendefekten, die Knochenneubildung und die Knochengewebezüchtung, **dadurch gekennzeichnet, dass** es ein Implantatmaterial nach einem der Ansprüche 1 bis 9 umfasst oder durch das Verfahren nach einem der Ansprüche 10 bis 20 erhalten wird.

## Claims

1. Implant material made of a hybrid material, said hybrid material comprising:
- a biodegradable polymer P soluble in at least one solvent S1 and insoluble in at least one solvent S different from the solvent S1 and an SiO₂ and CaO-based bioactive glass, optionally containing P₂O₅ and/or optionally doped with strontium,
**characterised in that** it comprises the superposition of:
- a porous portion having more than 90% by number, of pores of which the greatest dimension is greater than or equal to 100µm, and
- a dense portion (2, 20, 200, 2000, 20000) having more than 80% by number, of pores of which the greatest dimension is less than 50µm.

2. Implant material according to claim 1, **characterised in that** the dense portion volume/porous portion volume ratio is between 10/90 and 90/10.

3. Implant material according to claim 1 or 2, **characterised in that** the pores of the porous portion (1, 100, 1000, 10000) all have the same shape and the same dimensions.

4. Implant material according to claim 1 or 2, **characterised in that** the porous portion (10) has pores (30), of which the greatest dimension decreases from the implant base to the dense portion (20).

5. Implant material according to claim 1 or 2, **characterised in that** the porous portion has pores of which the greatest dimension increases from the implant base to the dense portion.

6. Implant material according to any one of the preceding claims, **characterised in that** the porous portion (1, 10, 100, 1000) and the dense portion (2, 20, 200, 2000) are in planar superposition.

7. Implant material according to any one of claims 1 to 5, **characterised in that** the porous portion (10000) and the dense portion (20000) are in concentric superposition.

8. Implant material according to any one of the preceding claims, **characterised in that** the pores of the porous portion have a spherical shape.

9. Implant material according to any one of claims 1 to 7, **characterised in that** the pores of the porous portion are in the form of polygons, preferably the form of squares.

10. Method for producing an implant material according to any one of claims 1 to 8, **characterised in that** it comprises the following steps:
a) selecting alkoxide precursors of an SiO₂ and CaO-based bioactive glass M, optionally containing P₂O₅ and/or optionally doped with strontium,
b) selecting a biodegradable polymer P which is soluble in at least one solvent S1 and insoluble in at least one solvent S, different from the solvent S1,
c) selecting microspheres of a porogenic agent A having diameters and sizes corresponding to the desired diameters and sizes of pores in the material constituting the implant to be produced, this porogenic agent A being:
- made of a polymer, insoluble in the at least one solvent S1 and soluble in the at least one solvent S,
the at least one solvent S, wherein the material of the biodegradable polymer P is insoluble, and the at least one solvent S, wherein the material of the porogenic agent A is soluble, being identical,
d) introducing microspheres of the porogenic agent A in a mould having the desired shape and size for the implant, these microspheres forming a compact stack corresponding to the size and to the shape of the pores to be obtained for the porous portion (1, 10, 100, 1000, 10000) of the implant material, and representing between 5% and 50% by volume, with respect to the total volume of the porogenic agent A - biodegradable polymer P - alkoxide precursors of the bioactive glass M mixture,
e) introducing the biodegradable polymer P in the alkoxide precursors of the bioactive glass M,
f) introducing the mixture obtained in step e) in the mould, in a quantity greater than the volume left vacant by the porogenic agent A beads, in order to obtain an implant material comprising the superposition of a porous region and of a dense region,
g) gelling of the mixture contained in the mould after step f),
h) demoulding the mixture obtained in step g),
i) removing porogenic agent A microspheres by washing with the solvent S.

11. Method for producing an implant material according to any one of claims 1 to 9, **characterised in that** it comprises the following steps:
a) selecting alkoxide precursors of an SiO₂ and CaO-based bioactive glass M, optionally containing P₂O₅ and/or optionally doped with strontium,
b) selecting a biodegradable polymer P which is soluble in at least one solvent S1 and insoluble in at least one solvent S, different from the solvent S1,
c) producing, by 3D printing, a preform (9), made of an polymer insoluble in the at least one solvent S1 and soluble in the at least one solvent S, this preform having the desired final shape and size for the pores (3, 30) in the porous portion (1, 10, 100, 1000, 10000) of the final implant and representing between 5% and 50% by volume of the total volume of the preform (9) - biodegradable polymer P - alkoxide precursors of the bioactive glass M mixture,
d) introducing the preform (9) in a mould having the desired shape and size of the final implant,
e) introducing the biodegradable polymer P in the alkoxide precursors of the bioactive glass M,
f) introducing the mixture obtained in step e) in the mould, in a quantity greater than the volume left vacant by the preform (9), in order to obtain an implant material comprising the superposition of a porous region and of a dense region,
g) gelling of the mixture contained in the mould after step f),
h) demoulding the mixture obtained in step g),
i) removing the preform (9) by washing with the solvent S.

12. Method according to claim 10 or 11, **characterised in that** the steps e) and/or f) are implemented before step d).

13. Method according to claim 11 or 12, **characterised in** the steps d), e) and f) are implemented simultaneously.

14. Method according to any one of claims 10 to 13, **characterised in that** in step d), the compact stack of microspheres or the preform (9) are placed so as to touch the side walls of the mould, by leaving a free space above the stack of microspheres or of the preform (9), whereby the porous portion (1, 10, 100, 1000) and the dense portion of the implant material (2, 20, 200, 2000) are in planar superposition.

15. Method according to any one of claims 10 to 13, **characterised in that** in step d), the compact stack of microspheres or the preform (9) are placed at the centre of the mould, by leaving a free space between the compact stack of microspheres or the side walls of the preform (9) and the side walls of the mould, whereby the porous portion (10000) and the dense portion of the implant material (20000) are in concentric superposition.

16. Method according to any one of claims 10 to 15, **characterised in that** the biodegradable polymer P is a biodegradable polymer soluble in at least one solvent S1 and insoluble in at least one solvent S selected from:
- bioresorbable polysaccharides, preferably selected from dextran, hyaluronic acid, agar, chitosan, alginic acid, sodium or potassium alginate, galactomannan, carrageenan, pectin,
- bioresorbable polyesters, preferably polyvinyl alcohol or poly(lactic acid),
- biodegradable synthetic polymers, preferably a glycol polyethylene, or poly(caprolactone),
- proteins, preferably gelatine or collagen,
and **in that** the material of the porogenic agent or of the preform (9) is a material selected from biodegradable polymers insoluble in the at least one solvent S1 and soluble in the at least one solvent S, preferably selected from C₁ to C₄ alkyl polymethacrylates, preferably methyl polymethacrylate or butyl polymethacrylate, polyurethane, polyglycolic acid, different forms of polylactic acids, lactic-coglycolic acid copolymers, polycaprolactone, fumarate polypropylene, paraffine and naphthalene, or acrylonitrile butadiene styrene (ABS),
the material of the porogenic agent A or of the preform (9) being different from the biodegradable polymer P.

17. Method according to any one of claims 10 to 16, **characterised in that** the biodegradable polymer P/bioactive glass M ratio by weight, is between 20/80 and 80/20 inclusive.

18. Method according to any one of claims 11 to 17, **characterised in that** the bioactive glass M is an SiO₂ and CaO-based glass, the biodegradable polymer P is gelatine, the material of the preform (9) is ABS and the solvent S is acetone.

19. Method according to any one of claims 10 and 12 to 17, **characterised in that** the bioactive glass M is an SiO₂ and CaO-based glass, the biodegradable polymer P is gelatine, the material of the porogenic agent A is methyl polymethacrylate and the solvent S is acetone.

20. Method according to any one of claims 10 to 19, **characterised in that** it comprises, in addition, to step f), a step of introducing a coupling agent, preferably an organoalkoxysilane compound, more preferably 3-glycidoxypropyltrimethoxysilane (GPTMS), even more preferably, 3-glycidoxypropyltriethoxysilane (GPTES).

21. Implant made of a hybrid material for the filling of bone defects, bone regeneration and tissue engineering of the bone, **characterised in that** it comprises an implant material according to any one of claims 1 to 9, or obtained by the method according to any one of claims 10 to 20.
